(19) Europäisches Patentamt
European Patent Office
Office européen des brevets

(11) **EP 3 811 917 B1**

(12) **EUROPEAN PATENT SPECIFICATION**

(45) Date of publication and mention
of the grant of the patent:
**03.12.2025  Bulletin 2025/49**

(51) International Patent Classification (IPC):
*A61F 13/537* (2006.01)   *D04H 3/08* (2006.01)
*D04H 3/147* (2012.01)   *D04H 3/16* (2006.01)

(21) Application number: **19204354.5**

(22) Date of filing: **21.10.2019**

(52) Cooperative Patent Classification (CPC):
**D04H 3/08; A61F 13/537; A61F 13/53747;
D04H 3/147; D04H 3/16**

(54) **ABSORBENT ARTICLE WITH SOFT ACQUISITION COMPONENT**

ABSORBIERENDER ARTIKEL MIT WEICHER ERFASSUNGSKOMPONENTE

ARTICLE ABSORBANT COMPORTANT UN COMPOSANT D'ACQUISITION SOUPLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.04.2021   Bulletin 2021/17**

(73) Proprietors:
• **Paul Hartmann AG**
  **89522 Heidenheim (DE)**
• **PFNonwovens Holding s.r.o.**
  **Hradcany**
  **11800 Praha (CZ)**
• **PFNonwovens Czech s.r.o.**
  **669 02 Znojmo (CZ)**

(72) Inventors:
• **MALOWANIEC, Krzysztof- Daniel**
  **89522 Heidenheim (DE)**
• **KESSELMEIER, Rüdiger**
  **89233 Burlafingen (DE)**
• **KASPÁRKOVÁ, Pavlína**
  **669 02 Znojmo (CZ)**
• **MECL, Zdenek**
  **67181 Nový Saldorf (CZ)**

(74) Representative: **Paul Hartmann AG**
  **Patents & Licensing**
  **Paul-Hartmann-Straße 12**
  **89522 Heidenheim (DE)**

(56) References cited:
**WO-A1-2014/022988     WO-A1-2015/066299
WO-A1-2018/059610**

**Description**

The invention relates to an absorbent article comprising an acquisition component, wherein the acquisition component comprises or consists of a nonwoven acquisition fabric containing multicomponent fibers.

Absorbent articles usually comprise an absorbent structure including an absorbent storage core for storing and retaining bodily fluids as well as an acquisition component for quickly acquiring the bodily fluids and distributing the fluids towards the storage core. A person skilled in the art will realize, for example, the advantages of carded nonwoven fabrics, i.e. nonwoven fabrics consisting of staple fibers, typically man made, which may or may not include multicomponent fibers. Such carded nonwoven fabrics until today are commonly used as acquisition components in an absorbent article for quickly taking up urine gushes and distributing the body liquids towards the absorbent storage core which typically contains superabsorbent material for storage and retention of the body fluids. Carding is a well-known process consisting of several production steps, where the fibers are first produced, then they are cut into short (staple) fibers, possibly treated, arranged to form a fibrous layer and then bonded together. Carded materials are produced from staple fibers and the high number of ends of these fibers located lengthwise and crosswise in a nonwoven layer may be undesired for certain applications.

Likewise, it is well known to use chemically modified cellulose fibers, e.g. intra-crosslinked cellulose fibers ("cellulose curly fibers") as an acquisition component typically disposed between the topsheet and the absorbent storage core of an absorbent article (as disclosed in e.g. WO9111163A1). Said cellulose curly fibers do not collapse upon wetting contrary to standard cellulose fibers and thereby safeguarding for an open structure which is able to repeatedly take up gushes of body fluids during use of an absorbent article. Both staple fiber carded nonwoven fabrics as well as cellulose curly fiber materials may work satisfactorily, but are costly to manufacture as fiber production and nonwoven production are separate processes. Therefore, a spunbond nonwoven acquisition component has been proposed (WO2018059610A1) which comprises at least two layers as follows:

- a first layer of filaments, wherein the first layer consists of continuous crimped bi-component filaments of an eccentric core/sheath structure, the filaments having a diameter in the range of 15 to 35 microns and exhibiting at least 3 crimps/cm, wherein the core of the filaments consists of a material having a higher melting point than the sheath,

- a second filament layer arranged in direct contact on the first layer, wherein the second layer of filaments comprises continuous crimped bi-component filaments of an eccentric core/sheath structure, the filaments having a diameter, which is smaller than the diameter of the filaments in the first layer and which is in the range of 10 to 20 microns and exhibiting at least 3 crimps/cm, wherein the core consists of a material having a higher melting point than the sheath.

The spunmelt process is an inline production process that forms the final spunbond nonwoven fabric from endless filaments in a single step. In particular, the advantages of multicomponent crimped or curled fibers with asymmetric (crimpable) cross-sections have been realized. It is well known in the industry, that certain combinations of polymers when arranged within a fiber in suitable arrangements, so called crimpable cross-sections, will provide fibers with crimping - even self-crimping immediately after spinning, or provide a certain level of latent crimping, that can be induced by activation, e.g. thermal activation. It is also well known that certain polymer composition combinations are better for softness and pliability and certain other polymer combinations are more suitable for good recovery. For example, the above mentioned patent application WO2018059610A1 describes the use of PET/PE compositions in an eccentric core/sheath arrangement to create a bulky layer providing a good combination of compressibility and recovery. WO2014022988 also discloses nonwoven made of crimped bicomponent fibers showing an eccentric core/sheath arrangement. WO2015066299 discloses nonwovens including both fines fibers and spunbond fibers. The spunbond fibers may be bicomponent fibers. The above mentioned and some other drawbacks of prior art are eliminated by an absorbent article comprising an absorbent storage core and an acquisition component, the acquisition component comprises a nonwoven acquisition fabric, wherein the nonwoven acquisition fabric is an air-through bonded nonwoven acquisition fabric, wherein the nonwoven acquisition fabric is a spunbond nonwoven, wherein the basis weight of the nonwoven acquisition fabric is 20-110 g/m$^2$, wherein the absorbent article comprises a length L1, the nonwoven acquisition fabric comprises a length L2, wherein L2<L1, the nonwoven acquisition fabric comprises filaments, the filaments comprise a first polymeric material and a second polymeric material, the second polymeric material having its melting point lower than the first polymeric material, wherein the first polymeric material and/or the second polymeric material consists of or comprises as the majority component polymeric material selected from the group consisting of polyesters, polyolefins, polylactic acid, polyester copolymers, polylactide copolymers and blends thereof; and wherein the first polymeric material is different from the second polymeric material, wherein the second polymeric material extends in the longitudinal direction of the filaments and forms at least a part of the surface of the filaments, wherein all components of the filaments are arranged across the cross-section of the filaments in a non-crimpable configuration, that is the center of gravity of surfaces formed by a component across the fiber cross-section is located in substantially the same location as the center of gravity of surfaces of each of the other components, wherein the filaments have a median fiber diameter of 5-50 microns, and the nonwoven acquisition fabric comprises filament-to-filament bonds formed of the second polymeric material wherein the nonwoven acquisition fabric has a structural softness as defined herein of at least 80 $m^4mm^2g^{-2}$, wherein

$$Structural\ softness = \frac{thickness}{basis\ weight} \times recovery \times \frac{compressibility}{basis\ weight} \times 10^6$$

wherein

- thickness is the thickness of the nonwoven structure in mm,
- basis weight is the basis weight of the nonwoven structure in grams per square meter,
- recovery is the ratio (Tr)/(Ts), wherein (Ts) is the initial thickness of the nonwoven structure under a pressure of 0,5 kPa and (Tr) is the recovered thickness of the nonwoven structure measured after a 2,5 kPa load has been applied and afterwards released,
- compressibility is in mm the difference between the initial thickness of the nonwoven structure and the thickness of the nonwoven structure under a pressure of 2,5 kPa, wherein all characteristics to be measured according to the test methods are defined herein below.

Preferably, the nonwoven acquisition fabric has a structural softness as defined herein of at least 100 $m^4mm^2g^{-2}$, preferably at least 110 $m^4mm^2g^{-2}$, more preferably at least 120 $m^4mm^2g^{-2}$, more preferably at least 130 $m^4mm^2g^{-2}$, more preferably at least 140 $m^4mm^2g^{-2}$, most preferably at least 150 $m^4mm^2g^{-2}$.

Also preferably, the filaments have a core/sheath structure, wherein the first polymeric material forms the core and the second polymeric material forms the sheath. The mass ratio of the first polymeric material to the second polymeric material is preferably 50:50 to 90:10.

The nonwoven acquisition fabric which the acquisition component of the absorbent article comprises or consists of has preferably a basis weight of 25-100 $g/m^2$, more preferably 30-90 $g/m^2$, in particular 40-80 $g/m^2$, in particular 50-70 $g/m^2$.

At a given fluid management performance, e.g. measured according to the rewet test defined herein below, it is preferred that the nonwoven acquisition fabric has a basis weight as low as possible. It is therefore preferred that the combined rewet-basis weight index FRW calculated as FRW = basis weight of the nonwoven acquisition fabric [$g/m^2$] * rewet [g] of the absorbent article is less than 110, more preferably less than 90, more preferably less than 80, even more preferably less than 70.

It is also advantageous, when the filaments have a median fibre diameter of at least 10 microns; preferably at least 15 microns; most preferably at least 20 microns, and at most 40 microns; most preferably at most 35 microns.

A suitable method of producing the nonwoven acquisition fabric comprising the steps

a) melting and feeding at least a first polymeric material and a second polymeric material having its melting point lower than the first polymeric material to nozzles of a spinning beam, wherein the nozzles are configured to form endless filaments preferably having all components arranged across the cross-section of the filaments in a non-crimpable configuration, wherein the second polymeric material extends in the longitudinal direction of the filament and forms at least a part of the surface of the filament, and the filament speed is within the range of 3000 and 5500 m/min,

b) cooling of the formed filaments by fluid medium having a temperature within the range of 10 to 90 °C and drawing the filaments with a draw down ratio within the range of 200-1300 to achieve a semi-stable crystalline state of at least the first polymeric material,

c) laying the filaments on a formation belt to form a nonwoven filamentary batt,

d) heating the nonwoven filamentary batt to a temperature within the range between 80 and 200°C to activate shrinkage of the nonwoven filamentary batt, such that at least the polymeric material is transformed to a more stable crystalline state.

The method may further comprise the step of pre-consolidation of the nonwoven filamentary batt after step c) and before step d), wherein the pre-consolidation is made by heating the filaments to a temperature within the range of 80 to 180 °C, preferably 90 °C to 150°C, most preferably 110°C to 140 °C to partially soften the polymeric material to provide bonds of polymeric material between the mutually crossing filaments.

In step b) the filaments may be cooled and drawn within a first zone with a fluid medium having a temperature within the range of 10 to 90 °C, preferably 15 to 80 °C, most preferably 15 to 70 °C, and then within a second zone with a fluid medium having a temperature within the range of 10 to 80 °C, preferably 15 to 70 °C, most preferably 15 to 45 °C.

Suitably, the heating of the nonwoven filamentary batt in step d) is provided by exposing the batt to air having the temperature within the range of 80 to 200 °C, preferably within the range of 100 to 160 °C, for a period of 20 to 5000 ms, preferably 30 to 3000 ms and most preferably 50 to 1000 ms. The air is preferably driven through and/or along the batt having initial speed within the range of 0,1 and 2,5 m/s, preferably within the range of 0,3 and 1,5 m/s.

Suitably, the nonwoven filamentary batt is heated in step d) such that it shrinks in the machine direction and cross direction by 20 % or less, preferably by 15 % or less, more preferably 13 % or less, more preferably 11% or less, most preferably 9 % or less, and increases its thickness by at least 20 %, preferably by at least 40 %, more preferably at least 60 %, most preferably by at least 100 %.

The nonwoven filamentary batt may be heated in step d) such that polymeric material softens to provide bonds of polymeric material between the mutually crossing filaments. Or, the nonwoven filamentary batt is heated after step d) such that polymeric material softens to provide bonds of polymeric material between the mutually crossing filaments. The heating after step d) to provide bonds of polymeric material (B) may be made using an omega drum bonding device, or a flat belt bonding device or a multiple drum bonder, and/or by driving air through and/or along the nonwoven filamentary batt for a time period of 200 to 20000 ms, preferably in between 200 and 15000 ms and most preferably in between 200 and 10000 ms, wherein the air has the temperature within the range of 100 °C to 250 °C, preferably 120 °C to 220 °C and initial velocity within the range of 0,2 and 4,0 m/s, preferably in between 0,4 and 1,8 m/s.

It is advantageous, when the draw down ratio is within the range of 300 - 800.

**Definitions**

The term "acquisition component" refers to a material layer in an absorbent article, comprising or consisting of a nonwoven fabric, disposed between the topsheet (if present as a separate component) and the absorbent storage core. This layer is designed to quickly acquire and/or distribute a fluid away from the body facing surface of the absorbent article and into the core. This layer is sometimes called a "wicking layer", "surge layer", "acquisition layer" or "distribution layer". Articles having an acquisition component consisting of only one sub-layer are known. Acquisition components having two sub-layers or more are also known. The acquisition component may - in addition to the nonwoven fabric - comprise other material layers such as apertured films or foams or the like. Ideally the acquisition component shall primarily pull the fluid quickly away from the body facing surface or the topsheet (if present) and distribute the fluid in the direction towards the core and also in other directions throughout the acquisition component. The acquisition component should lower the tendency of fluid travel back from the core towards the topsheet/body facing surface, i.e. to lower or prevent rewetting of the topsheet/body facing surface. The acquisition component typically does not comprise superabsorbent material. In the following, the term "acquisition component" will be used to designate the material layer present between the body facing surface, i.e. the topsheet if present, and the absorbent storage core providing these acquisition and distribution functions, irrespective of the number of sub-layers forming this layer.

As used herein, the term "absorbent storage core" refers to the component of the absorbent article that is primarily responsible for retaining and storing body fluids. As such, the absorbent storage core typically does not include the topsheet or backsheet of the absorbent article and is a component separate from the acquisition component.

The term "batt" refers to materials in the form of filaments that are found in the state prior to bonding, a process that can be performed in various ways, for example, air-through-bonding, calendering etc. The "batt" consists of individual filaments between which a fixed mutual bond is usually not yet formed even though the filaments may be pre-bonded / pre-consolidated in certain ways, where this pre-consolidation may occur during or shortly after the laying of the filaments in the spunlaying process. This pre-consolidation, however, still permits a substantial number of the filaments to be freely moveable such that they can be repositioned. The above mentioned "batt" may consist of several strata created by the deposition of filaments from several spinning beams in the spunlaying process.

The term "filament" refers to a principally endless fiber, while the term "staple fiber" refers to a fiber which has been cut to a defined length.

The term "filament to filament bonds" refers to bonds which connect usually two filaments in an area, in which the filaments cross each other or locally meet or abut on each other. The bonds may connect more than two filaments or may connect two parts of the same filament.

The term "monocomponent filament" refers to a filament formed of a single polymer or polymer blend, as distinguished from a bicomponent or multicomponent filament. "Multicomponent fiber or filament" refers to a fiber or filament having a cross-section comprising more than one discrete section, where each of these sections comprises a different polymer component, or a different blend of polymer components, or polymer component and blend of polymer components. The term "multicomponent fiber / filament" includes, but is not limited to, "bicomponent fiber / filament." The different components of multicomponent fibers are arranged in substantially distinct regions across the cross-section of the fiber and extend continuously along the length of the fiber. A multicomponent fiber may have an overall cross-section divided into subsections of the differing components of any shape or arrangement, including, for example, coaxial subsections, core-and-sheath subsections, side-by-side subsections, radial subsections, islands-in-the-sea subsections, etc.

A bicomponent filament having a "core/sheath structure" has a cross-section comprising two discrete sections each of which is comprised of a polymer or polymer blend, wherein the sheath polymer or polymer blend component is enclosed around the core polymer or polymer blend component.

"Fiber diameter" is expressed in units of $\mu$m. The terms "grams of fiber per 9000 m" (denier or den) or "grams of fiber per 10000 m" (dTex) are used to describe the fineness or coarseness of fibers, which are related to the diameter (when assumed to be circular) by the density of the employed material(s). "Film" - means a skin-like or membrane-like layer of material formed of one or more polymers or polymer blends, which does not have a form consisting predominately of a web-like structure of consolidated polymer fibers and/or other fibers.

"Machine direction" (MD) - with respect to the production of a nonwoven web material and the nonwoven web material, machine direction (MD) refers to the direction along the web material substantially parallel to the direction of forward travel of the web material through the production line on which the web material is manufactured.

"Cross direction" (CD) - with respect to the production of a nonwoven web material and the nonwoven web material, cross direction (CD) refers to the direction along the web material substantially perpendicular to the direction of forward travel of the web material through the production line on which the web material is manufactured.

A "nonwoven" or "nonwoven fabric" or "nonwoven web" is a manufactured sheet or web of directionally or randomly oriented fibers which are first formed into a batt and then consolidated together by friction, cohesion, or adhesion and bonded thermally (e.g. air-through-bonding, calander-bonding, ultrasonic bonding, etc.), chemically (e.g. using glue), mechanically (e.g. hydro-entanglement, etc.) or by combination thereof. The term does not include fabrics which are woven, knitted, or stitch-bonded with yarns or filaments. The fibers may be of natural or man-made origin and may be stapled or continuous ("endless") filaments or be formed in-situ. Commercially available fibers have diameters ranging from less than about 0,001 mm to more than about 0,2 mm, and come in several different forms: short fibers (known as staple or chopped fibers), continuous single fibers (filaments or monofilaments), untwisted bundles of continuous filaments (tow), and twisted bundles of continuous filaments (yarn). Nonwoven fabrics can be formed by many processes including, but not limited to, meltblowing, spunbonding, spunmelting, solvent-spinning, electro-spinning, carding, film fibrillation, melt-film fibrillation, airlaying, dry-laying, wet-laying with staple fibers, and combinations of these processes as known in the art. The basis weight of nonwoven fabrics is usually expressed in grams per square meter ($g/m^2$).

As used herein, the term "layer" refers to a component or element having its extension essentially in the plane (x/y-direction), i.e. its z-direction extension is much smaller than its x/y direction extension. In the context of a nonwoven fabric comprising or consisting of filaments a "layer" may be in the form of a plurality of fibers made on a single beam or on two or more consecutive beams, which produce substantially the same fibers. For example, two consecutively arranged spunbond beams with substantially the same settings and polymer compositions can together produce a single layer. In contrast, for example, two spunbond beams, where one produces monocomponent fibers and the other bicomponent fibers, will form two distinct layers. The composition of a layer can be determined either by knowing the individual settings and components of the resin (polymer) composition used to form the layer, or by analyzing the nonwoven itself, using, for example, optical or SEM microscopy or by analyzing the composition used to make the fibers of the layer using DSC or NMR methods.

The "spunbond" process is a nonwoven manufacturing system involving the direct conversion of a polymer into continuous ("endless") filaments, integrated with the conversion of the filaments into a random arrangement of laid filaments forming a nonwoven batt that is subsequently bonded to form a nonwoven fabric. The nonwoven acquisition fabric which the acquisition component of the absorbent article of the present invention comprises or consists of a spunbond nonwoven. Bonding process can be performed in various ways, for example, air-through-bonding, calendering etc. "Activation" herein refers to the process, whereby fibers or filaments or fiber structures being in a semistable state (for example not being crystallized in the lowest possible energy state) are heated and then slowly cooled so, that the semistable state changes to some other more stable state (for example a different crystallization phase).

The term "crimpable cross-section" herein refers to multicomponent fibers, where components, i.e. the polymeric materials making up the fibers, with different shrinkage properties are arranged across the cross-section so, that when heated to or above the activation temperature and then slowly cooled down, the fibers crimp, which causes these fibers to follow the vectors of the shrinkage forces.

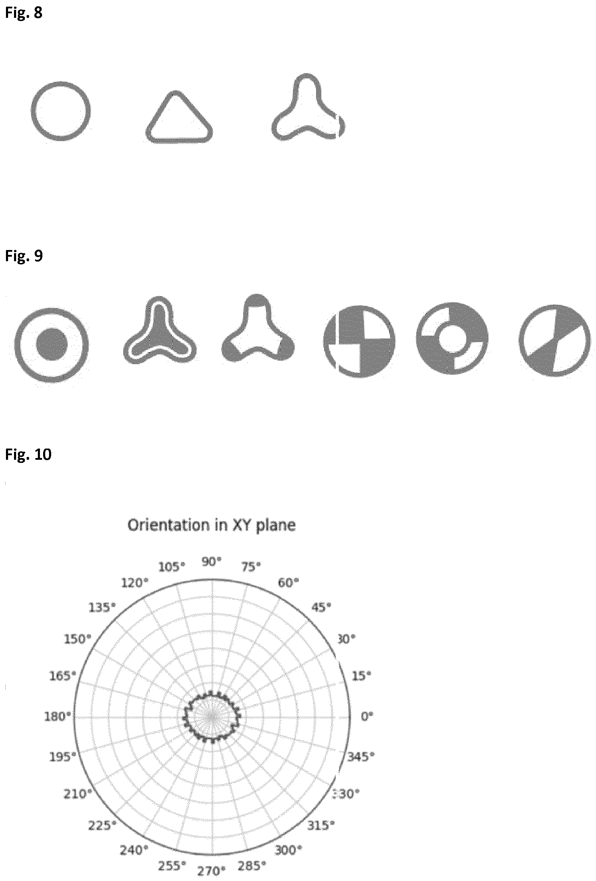

Thereby, when the fiber is released, it creates a so-called helical crimp, although when contained within a fiber layer the mutual adhesion of the fibers does not permit the creation of ideal helixes. For a multicomponent fiber, we can determine the center of mass for each individual component in the fiber cross-section (considering their areas/positions in the cross-section). Not to be bound by a theory, we believe that when the centers of gravity of all areas of each of the components are substantially at the same point, the fiber is non-crimpable. For example, for a round bicomponent fiber with centric core/sheath structure the center of mass is in the center of the cross-section (see the Fig. 9).

The term "compressibility" herein refers to the distance in millimeters (mm) by which the nonwoven is compressed by a load defined by a "resilience" measurement as defined herein below.

The term "spinneret capillary density [1000/m]" herein refers to the number of capillaries placed on the spinneret per 1 m distance in the CD.

The term "filament speed" herein refers to a number calculated from the fiber diameter, the throughput and the polymer density of the filament.

The term "draw down ratio" herein refers to a number calculated by dividing the capillary cross-section area by the filament cross-section area. The measured fiber fineness based on its apparent diameter is used to calculate the filament cross-section area. Other non-round cross-sections cannot be calculated in this way, thus in such cases the analysis of SEM images showing the actual cross-section is necessary.

The term "cooling air / polymer ratio" herein refers to a calculated number out of the cooling air mass flow divided by the polymer mass flow.

As used herein, the term "absorbent article" refers to wearable devices, which absorb and/or contain liquid, and more specifically, refers to devices, which are placed against or in proximity to the body of the wearer to absorb and contain the various exudates discharged from the body. Suitable non-limiting examples include diapers, prefastened diapers, reclosable diapers, training pants, incontinence pants or pull-on garments, incontinence pads, incontinence slips, and feminine care products such as sanitary napkins.

**Brief description of the drawings**

Some of the preferred embodiments of the invention will be described in more detail with reference to the accompanying schematic drawings, which show

| | |
|---|---|
| Fig. 1: | A top view of the body facing surface of a T-shape incontinence slip |
| Fig. 2: | shows a sectional view of the diaper as per figure 1, by way of the section plane A-A |
| Fig. 3: | A schematic illustration of the incontinence slip of figure 1 in a worn state |
| Fig. 4: | A top view of the body facing surface of an H-shape incontinence slip |
| Fig. 5: | shows a sectional view of the incontinence slip as per figure 4, by way of the section plane B-B |
| Fig. 6: | A top view of the body facing surface of an incontinence pad |
| Fig. 7: | A top view of the body facing surface of an incontinence pant |
| Fig. 8: | Filament shapes |
| Fig. 9: | Examples of non-crimpable cross-section |
| Fig. 10: | Omnidirectionality of filaments in a nonwoven acquisition fabric |
| Fig.11: | Shrinkage of example 2F in contrast to shrinkage of example 4 |
| Fig.12: | SEM microscopy photo of core/sheath fiber cross-section before and after activation |
| Fig.13 a - c: | Examples of filament routes in a nonwoven acquisition fabric |
| Fig. 14: | Micrographs of fiber layers with different crimp level |
| Fig. 15: | Fabric nonwoven cross-section - example 7C (crimped fibers) |
| Fig. 16: | Fabric nonwoven cross-section - examples 5A+5D |
| Fig.17: | Fabric nonwoven cross-section |
| Fig. 18A and 18B: | Production lines suitable for manufacturing a nonwoven acquisition fabric |
| Fig. 19 a-c: | "length of the filament to the length of the fabric ratio" - illustrative image to method b) |
| Fig. 20 a, b: | Test method set up for rewet measurements |

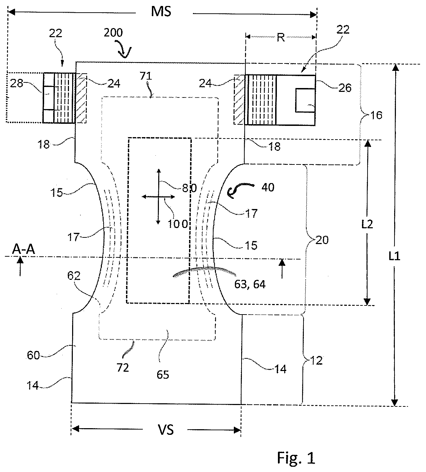

The figures 1-3, not to scale but schematically, show an absorbent incontinence slip 200 according to the invention, in the so-called T-shape, configured for adults. The incontinence slip 200 comprises a main part 40 having an absorbent storage core 65 that retains and stores bodily fluids. The absorbent storage core 65 preferably comprises a homogeneous mixture of cellulose fibers and superabsorbent polymer particles (SAP). An acquisition component 63, in the present embodiment consisting of a nonwoven acquisition fabric 64, is disposed on top of the absorbent storage core 65. The assembly of the absorbent storage core 65 and the nonwoven acquisition fabric 64 is disposed between two planar sheet-like materials, specifically a liquid-permeable cover layer 60 (topsheet) and a liquid-impermeable back layer 62 (backsheet) of the diaper main part 40.

As to the absorbent incontinence slip 200, a longitudinal direction 80 and a transverse direction 100 are distinguishable, wherein the latter in the worn state of the slip 200 corresponds to the circumferential direction of the hip of the user. The main part 40 comprises a front region 12 having forward lateral longitudinal peripheries 14, a back region 16 having rearward lateral longitudinal peripheries 18, and disposed therebetween a crotch region 20. The main part 40, so as to be adjacent to a respective longitudinal periphery 15 of the crotch region 20, has in each case one elasticized portion 17, consequently an elasticized leg opening portion. These elasticized leg opening portions are formed by elastic threads which run between the top sheet 60 and the back sheet 62 and in the pre-tensioned state are fixed to the top sheet 60 and the back sheet 62 and which are curved in an arcuate manner.

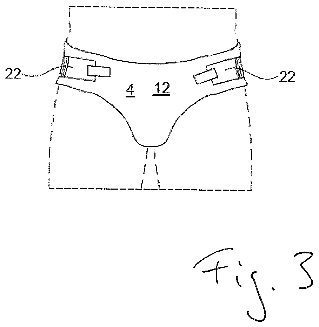

In the case of the illustrated embodiment of the T-shape incontinence slip 200, rear side panels 22 which, when unfolded, in the transverse direction 100 extend laterally over a width of R and beyond the rearward lateral longitudinal peripheries 18 are provided only in the back region 16 of the main part 40. Said rear side panels 22 in the region of the rearward lateral longitudinal peripheries 18 being non-releasably joined to the back region 16 of the main part 40 in an overlap region 24. Preferably, prior to use, both rear side panels 22 are folded onto itself along fold lines running in a longitudinal direction. For illustrative purposes the right rearward side panel is shown in its unfolded form while the left rearward side panel is still folded onto itself. Upon unfolding of the left rearward side section (illustrated by dotted line of left rearward side section), the maximum span MS provides for a ratio of the maximum span MS of the incontinence slip 200 to a front span VS of the incontinence slip of preferably 1,3 - 2,8, in particular 1,4 - 2,7, further in particular 1,5 - 2,6, further in particular 1,6 - 2,5. The span is defined as the maximum transverse extension of the region of concern of the incontinence article in the unfolded, flattened and unstretched state measured in mm. The rear side panels 22 in the region of the free end 26 thereof in the transverse direction 100 have in each case at least one closure means 28. The closure means 28 is configured in the form of a preferably rectangular tab and is folded onto the body facing side of the rearward side panel. The closure means can be opened, that is to say unfolded again, in the use situation, so as to place the absorbent incontinence slip 200 on a user, wherein the side panels 22 are brought to overlap with the front region 12 of the main part 40 and the closure means are fastened so as to releasably adhere to the external side of the front part 12 of the main part 40 (schematically illustrated in figure 3).

The incontinence slip 200 comprises a length L1, the nonwoven acquisition fabric comprises a length L2, wherein L2<L1. The nonwoven acquisition fabric 64 is disposed at a distance in the longitudinal direction 80 from both the back end of the incontinence slip 200 and the front end of the incontinence slip 200. More over, the nonwoven acquisition fabric 64 is disposed at a distance in the longitudinal direction 80 of the absorbent article from both the back end 71 of the absorbent storage core 65 and the front end 72 of the absorbent storage core 65.

It should be noted here that, in the present application, any dimensional information relating to transverse extension, longitudinal extension of sections or of any component of the absorbent article is determined in the evenly spread out state of the flat materials constituting the article, so that the article in question can be brought into the even configuration depicted in the figures, unless another indication of a different state is given. If, for example, the article has been elasticized by means of thread-like elasticizers in the so-called "stretchbond process", the surface materials, as indicated in the figures, are considered as extended as they are supplied by the manufacturer as flat materials or can subsequently be spread out until their natural initial extension without elasticizer and placed on a flat surface. In this flat surface, the transverse stretches, longitudinal stretches or dimensions are then determined. This condition results naturally from inextensible chassis materials based on nonwoven or film or nonwoven-film composite.

Deviating from this, all dimensions of the absorbent article with regard to the transverse extension, insofar as they comprise the elastic area in the transverse direction, e.g. of the side panels of an H-shape incontinence slip or a T-shape incontinence slip, - namely the maximum span or the side panel width - are carried out in the transverse direction only in the expanded and unfolded but unstretched state. Measurements in the unstretched state reflect the state in which the user perceives the product when first taking it by hand and removing it from the packaging after unfolding the absorbent article. In particular, no transverse tensile forces are exerted on the side sections, as they typically occur when the diaper is applied to the user.

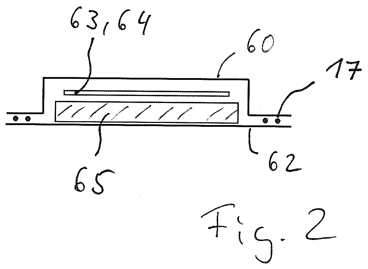

Figure 2 is showing the arrangement of topsheet 60, backsheet 62, elasticized portion 17, nonwoven acquisition fabric 64 constituting the acquisition component 63, absorbent storage core 65 consisting of a single layer of cellulose fibers mixed with superabsorbent polymer particles.

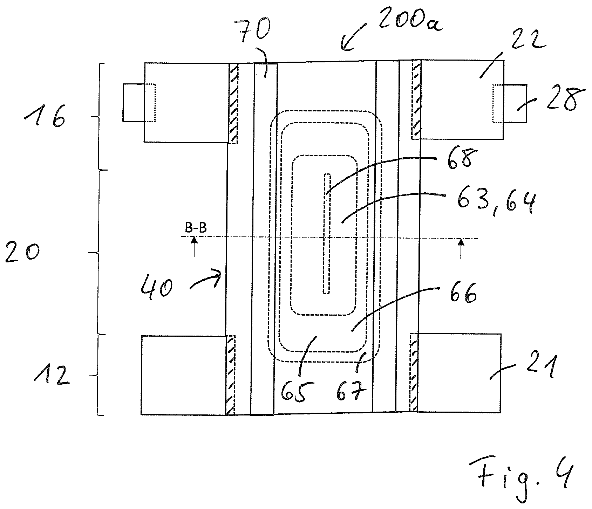

Figure 4 shows a schematic top view of the body facing surface of an H-shape absorbent incontinence slip 200a in a state of having just been unfolded. Same components illustrated in Figures 1-3 as well have been given the same reference numbers. Unlike the T-shape absorbent incontinence slip illustrated above, the H-shape absorbent incontinence slip shows both rear side panels 22 and front side panels 21. Rear side panels 22 and front side panels 21 have been separately attached to respective side edges of the main part 40. So as to place the H-shape absorbent incontinence slip 200a on a user, the rear side panels 22 are brought to overlap with the front side panels 21 or with the front region 12 of the main part 40 and the closure means 28 (illustrated in their unfolded form, "ready-to-use") are fastened so as to releasably adhere to the external side of the front side panels 21 or the front part 12 of the main part 40. The absorbent storage core 65 in this case consists of an upper layer 66 and a lower layer 67, wherein the upper layer 66 is provided with a first area and a second area, the basis weight of the absorbent material of the first area is different from the basis weight of the absorbent material of the second area. In particular the second area consists of a channel 68, i.e. an area free of absorbent material, meaning the basis weight of the absorbent material in the channel is 0 g/m$^2$. In the embodiment of Figure 4 the incontinence slip 200a is provided with barrier cuffs 70 consisting of an SMS nonwoven web material which is liquid impermeable under normal in-use conditions.

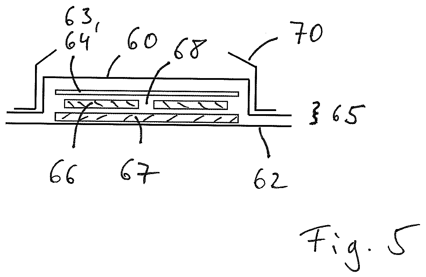

Figure 5 is showing the arrangement of topsheet 60, barrier cuffs 70 backsheet 62, nonwoven acquisition fabric 64 constituting the acquisition component 63, absorbent storage core 65 consisting of upper layer 66 and lower layer 67, the upper layer provided with a channel 68.

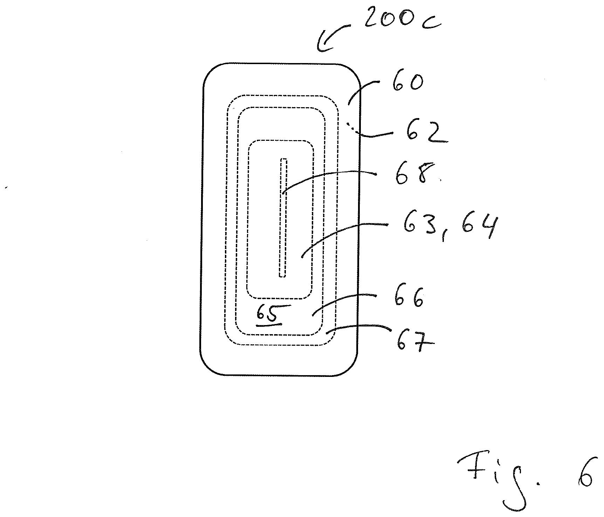

Figure 6 shows another embodiment of an absorbent article, configured as an incontinence pad 200c. Same components as far as illustrated in Figures 1-3 as well have been given the same reference numbers. As can be seen, the absorbent storage component 65 is designed similar to the absorbent storage component of the above illustrated H-shape incontinence slip. Meaning the absorbent storage core 65 consisting of upper layer 66 and lower layer 67, the upper layer provided with a channel 68. A nonwoven acquisition fabric 64 constituting the acquisition component 63 is disposed between the topsheet 60 and the absorbent storage core 65. The backsheet 62 together with the topsheet 60 serves to envelope the assembly of nonwoven acquisition fabric 64 and absorbent storage core 65.

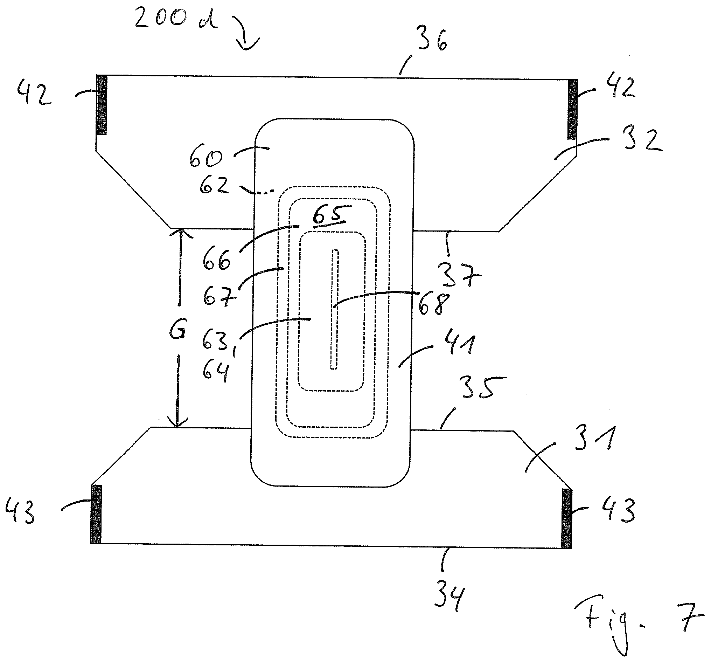

Figure 7 shows another embodiment of an absorbent article, configured as an incontinence pant, in particular it shows a schematic top view of the body facing surface of an H-shape incontinence pant 200d, however prior to bonding side edges regions 43 of an elastic front body panel 31 to respective side edge regions 42 of an elastic rear body panel 32 to form a waist opening and two leg openings similar to normal underware. The same components as far as illustrated in Figures 1-3 as well have been given the same reference numbers. The front body panel 31 comprising a terminal waist edge 34 and a terminal crotch end 35 and the rear body panel 32 comprising a terminal waist edge 36 and a terminal crotch edge 37, wherein said terminal crotch edge 37 of said rear body panel is longitudinally spaced from and forms a gap G with said terminal crotch edge 35 of said front body panel 31, and wherein an absorbent insert 41 bridges said gap G and is connected to the body facing surface of said front 31 and rear body panels 32 by e.g. hotmelt adhesive. The absorbent storage component 65 is designed similar to the absorbent storage component of the above illustrated H-shape incontinence slip. Meaning the absorbent storage core 65 consisting of upper layer 66 and lower layer 67, the upper layer provided with a channel 68. A nonwoven acquisition fabric 64 constituting the acquisition component 63 is disposed between the topsheet 60 and the absorbent storage core 65. The backsheet 62 together with the topsheet 60 serves to envelope the assembly of nonwoven acquisition fabric 64 and absorbent storage core 65.

In the following the nonwoven acquisition fabric for use as the acquisition component of an absorbent article of the present invention is further characterized. According to a preferred embodiment of the invention, the nonwoven acquisition fabric which the acquisition component comprises or consists of comprises or consists of at least one layer, preferably 1-4 layers, more preferably two layers, most preferably one layer. Preferably said layer or layers consist of filaments, the filaments comprise a first polymeric material and a second polymeric material, the second polymeric material having its melting point lower than the first polymeric material, wherein the second polymeric material extends in the longitudinal direction of the filaments and forms at least a part of the surface of the filaments, and the nonwoven fabric comprises filament-to-filament bonds formed of the second polymeric material. The nonwoven acquisition fabric is an air-through bonded nonwoven fabric.

In another embodiment, the nonwoven acquisition fabric does not contain any staple fibers. In another embodiment, the nonwoven acquisition fabric does not contain any meltblown fibers.

The nonwoven acquisition fabric is formed mainly or entirely from endless filaments with a non-crimpable cross-section. The fibers are multicomponent, preferably bicomponent. Not to be bound by a theory, we believe that when the center of gravity of surfaces formed by a component across the fiber cross-section is located in substantially the same location as the center of gravity of surfaces of each of the other components, the cross-section is non-crimpable.

For example, the nonwoven acquisition fabric can comprise mainly endless filaments with a round cross-section, trilobal cross-section, star cross-section, etc. (Fig. 8). A person skilled in the art will realize many possible shapes of the fiber cross-section that will substantially neither crimp when cooled, nor involve latent crimping, which may, however, be activated by heating and subsequent cooling of the fibers.

For example, endless filaments can be multicomponent filaments, where the component layout in the cross-section is core/sheath (concentric), segmented pie or any other layout with the centre of gravity of component areas in one location within the filament cross-section (Fig. 9).

Preferably, the nonwoven acquisition fabric is formed from bicomponent core/sheath filaments with a round or trilobal shape.

Preferably, the endless filaments are formed from two or more components, where one component brings a certain level of strength and rigidity that is necessary for the recovery feature and the other component brings softness and also is able to maintain a cohesive structure by forming bonds between the individual filaments. For example, the first component can be chosen from a group of polyesters (e.g. from aromatic polyesters such as polyethylene terephthalate (PET), or from aliphatic polyesters such as polylactic acid (PLA)), polyamides, polyurethanes or their copolymers or suitable blends. It is within the scope of the invention that the first component consists or consists essentially of a plastic of the group of polyesters that also includes polyester copolymers (coPET) or polylactide copolymers (COPLA). Preferably polyethylene terephthalate (PET) or polylactic acid (PLA) is used as the polyester.

For example, the second component can be chosen from a group of polyolefins (i.e. polypropylene (PP) or polyethylene (PE)), low-melting polymers, copolymers or blends of suitable polymers. It is within the scope of the invention that the second component consists or consists essentially of a plastic of the group of polyesters that also includes polyester copolymers (coPET) or polylactide copolymers (COPLA). Preferably polyethylene (PE) is used as the polyolefin.

The preferred combination of components for the bicomponent filaments in the nonwoven layer according to the invention are PET / PE, PET / PP, PET / CoPET, PLA / COPLA, PLA / PE and PLA / PP.

The preferred bicomponent filaments have the ratio of the mass of the first component to the mass of the second component from 50:50 to 90:10.

In another embodiment, the components can also contain additives to modify the filament properties. For example, the core can contain a color pigment or, for example, a nucleating agent. A person skilled in the art will understand that special combinations of nucleating agents can be found that can change the polymer crystallization and shrinkage behavior up to a significant level (as for example shown by Gajanan in patent US5753736, filed in 1995). On the other hand, for example, simple titanium dioxide, which is often used as a whitening coloring agent, will cause only an insignificant change in the polymer behavior that can be, in case of need, easily offset by a slight adjustment of the process conditions.

The sheath can contain, for example, a color pigment or a surface modifier (to attain, for example, a silky touch and feel quality). A person skilled in the art will realise many other options based on requirements of specific applications.

Some polymers used for nonwoven fiber production may be inherently hydrophobic, and for certain applications they may be surface treated or coated with various agents to render them hydrophilic. A surface coating may include a surfactant coating. One such surfactant coating is available from Schill & Silacher GmbH, Böblingen, Germany, under the Tradename Silastol PHP 90. Another way to produce nonwovens with durably hydrophilic coatings, is via applying a hydrophilic monomer and a radical polymerization initiator onto the nonwoven, and conducting a polymerization activated via UV light resulting in monomer chemically bound to the surface of the nonwoven as described in US2005/0159720A1. Another way to produce hydrophilic nonwovens made predominantly from hydrophobic polymers such as polyolefins is to add hydrophilic additives into the melt prior to extrusion. Another way to produce nonwovens with durably hydrophilic coatings is to coat the nonwoven with hydrophilic nanoparticles as described in US7112621B1 and in WO02/064877A1. In some cases, the nonwoven web surface can be pre-treated with high energy treatment (corona, plasma) prior to application of nanoparticle coatings. High energy pre-treatment typically temporarily increases the surface energy of a low surface energy surface (such as PP) and thus enables better wetting of a nonwoven by the nanoparticle dispersion in water. A nonwoven also may include other types of surface coating. In one example, the surface coating may include a fiber surface modifying agent that reduces surface friction and enhances tactile lubricity. *Preferred fiber surface modifying agents are described in* US6632385B1 *and* US6803103B1 *and* US2006/0057921A1.

In another embodiment, the components can also contain a certain amount of different polymers. For example the first component (e.g. core) can contain a certain small amount of the second component (e.g. sheath) polymer or polymers, or vice versa the second component (e.g. sheath) can contain, for example, a small amount of second component (e.g. core) polymer or polymers. A person skilled in the art will know that a certain content level can be found for exact polymer combinations. For example Moore teaches (US2012088424), that blend up to 10% of polypropylene to polyester will provide stable fibers.

Not to be bound by a theory, we believe that an important topic for the formation of the fabric with the desired properties is achieved by the combination of two components. Firstly, the component of the filament forming the nonwoven acquisition fabric, according to the invention, forming, for example, the core, comprises of polymer A that is able to shrink under certain conditions. During the fiber formation process - especially during cooling and drawing - polymer A is designed to be able to undergo changes upon future activation. For example, polymer A is set to a semistable state (for example, not crystallized in the lowest possible energy state) and then during activation it is heated and then slowly cooled so that the semistable state changes to some other more stable state (for example different crystallization phase with a lower volume). This change results in inner shrinkage forces that we believe have their vector in the direction of the fiber center line.

Preferably, the fiber diameters within the nonwoven acquisition fabric are in the millimeter and/or submillimeter range; in general they are omnidirectional (see Fig. 10) and contact each other so that the free parts between them are also generally in the millimeter and/or submillimeter range. The cohesion between the fibers acts against the inner force vector and forms the first resistance point against it. This resistance point can be also called threshold resistance point against structural shrinkage. For example, when one fiber is set to the proper state and undergoes activation, it can form, for example, irregular bows or waves in all 3 dimensions. In contrast, a fiber limited by the surrounding structure of its neighboring fibers does not have such freedom.

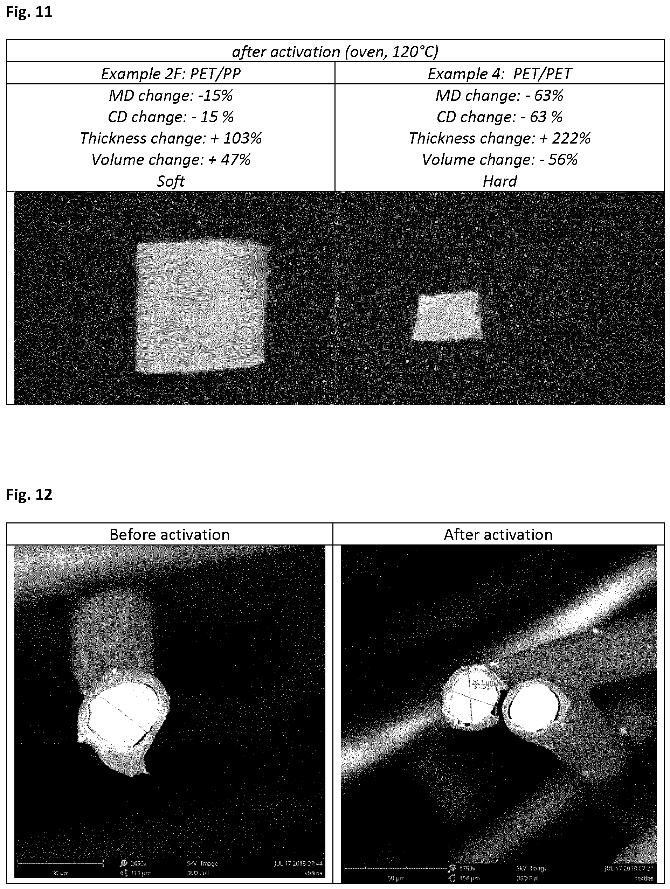

The nonwoven acquisition fabric of the absorbent article is preferably formed from bicomponent filaments, where the second component comprises of polymer B that has a lower melting point and preferably also provides other desired properties such as softness, pleasant touch and feel enhancing properties, etc. Polymeric material A and polymeric material B should differ in their shrinkage characteristics, preferably the polymeric material B (preferably the material forming the sheath of the filament) has a lower shrinkage potential than the polymeric material A (preferably forming the core of the filament). The result is differing shrinkage forces acting within the two adjacent polymeric materials. Not to be bound by a theory, we believe that the polymeric material A and the polymeric material B always have different characteristics, so that the vectors of the inner shrinkage forces are never the same at the same point in time. This inhomogeneity in forces forms the second threshold resistance point against shrinkage. This resistance point can also be defined as a threshold resistance point against fiber shrinkage. For example, by comparing the behavior of the layer formed from a monocomponent filament (e.g. PET) and a layer formed under the same conditions from bicomponent filaments (e.g PET/PP), we can find a significant difference. Both samples of the same size, produced under the same conditions, were exposed to the activation temperature of 120 °C for the same time. Mono PET shrunk to a small planar object, while, in contrast, the PET/PP structure increased its volume (small decrease in CD and MD plus a large increase in the z-direction - see Table 1 and Fig. 11).

*Table 1:*

| after activation (oven, 120°C) | |
| --- | --- |
| *Example 2F: PET/PP* | *Example 4: PET/PET* |
| *MD change: -15%* <br> *CD change: - 15 %* <br> *Thickness change: + 103%* <br> *Volume change: + 47%* <br> *Soft* | *MD change: - 63%* <br> *CD change: - 63 %* <br> *Thickness change: + 222%* <br> *Volume change: - 56%* <br> *Hard* |

Preferably, the batt layer undergoing activation provides a CD or MD shrinkage of at most 20%, preferably of at most 15%, preferably maximum 13%, more preferably maximum 11%, more preferably maximum 9%.

Preferably the batt layer undergoing activation provides a z-direction increase of at least 20%, preferably of at least 40%, preferably of at least 60%, preferably of at least 80%, more preferably of at least 100%.

With considerable simplification, we can say that a batt shrinkage level can be estimated by the single fiber shrinkage level.

Preferably, the batt layer undergoing activation provides a positive volume change; preferably the volume change is higher than 10%, preferably higher than 15%, more preferably higher than 20%.

A person skilled in the art will know that a sensitive process such as spunbonding can also be influenced by various other conditions that can also induce certain opposing forces acting against both fiber and structure shrinkage.

It is well known in the industry that certain combinations of polymers with different shrinkage levels arranged in a so-called crimpable cross-section provide for so-called crimping. This can be either immediate self-crimping or latent crimping, where the fibers have to be activated in order to exhibit crimps (for example, through thermal activation). Fibers with crimpable cross-sections provide regular crimps forming a so-called helical crimp. With considerable simplification, we can say that a fiber having a crimpable cross-section tends to bend in the direction towards the component with higher shrinkage, which causes a substantially uniform helical crimp. In other words, the crimpable cross-section causes the regular shifting of the inner force vectors of the first and second components towards each other. Not to be bound by a theory, we believe that the regularity of the shift is the main reason for the regularity of the crimp on the free single fiber. In contrast, and not to be bound by a theory, on fibers that have a non-crimpable cross-section, we believe that the inner shrinkage force vectors of the first and second component do not provide any regular shift between each other, and thereby the fiber forms irregular bows or waves in arbitrary directions. With considerable simplification we can say that the fiber does not have a uniform tendency to bend towards a specific part of its cross-section or periphery, which results in its irregular final shape. After activation, the fiber cross-section remains substantially non-crimpable, see Fig. 12.

Not to be bound by a theory, we believe that when the inner shrinkage force is weak and is unable to overcome the threshold fiber resistance point of the opposing forces, the fabric will remain unchanged. When the inner shrinkage forces are strong enough and able to overcome all the threshold MD/CD resistance points of the opposing forces, the fabric shrinks according to the MD/CD ratio and forms a flat structure. When the inner shrinkage force is just strong enough to overcome the threshold fiber shrinkage resistance points but not strong enough to overcome the threshold structural MD/CD shrinkage resistance points, the fibers will bend out like springs in various directions, facing the lowest structural resistance mainly from the z-direction, and will form the desired bulky structure. Thus, a person skilled in the art will understand that the desired inner fiber shrinkage force will be higher than the inner resistance point of the fiber, yet lower than the threshold structural MD/CD shrinkage resistance point.

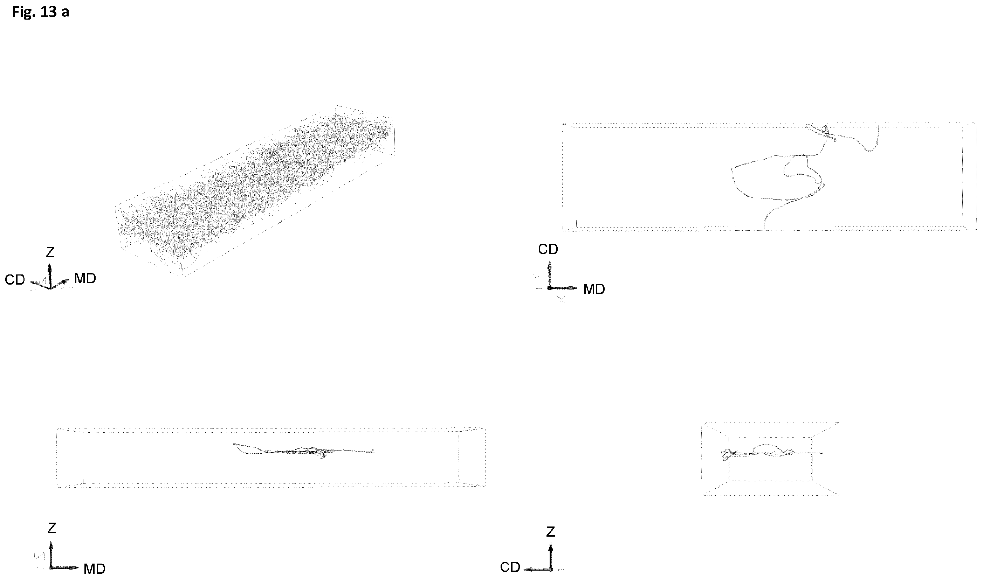

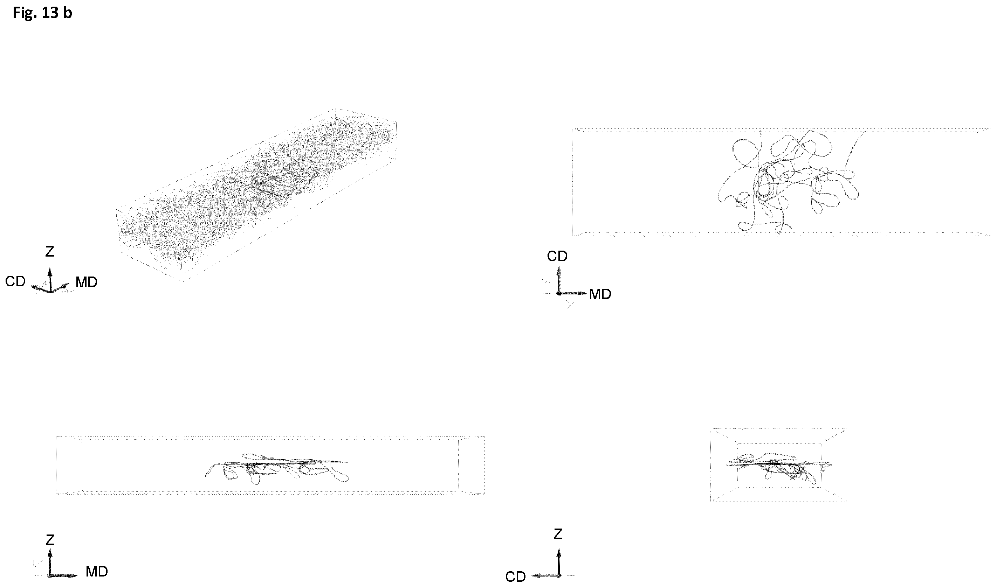

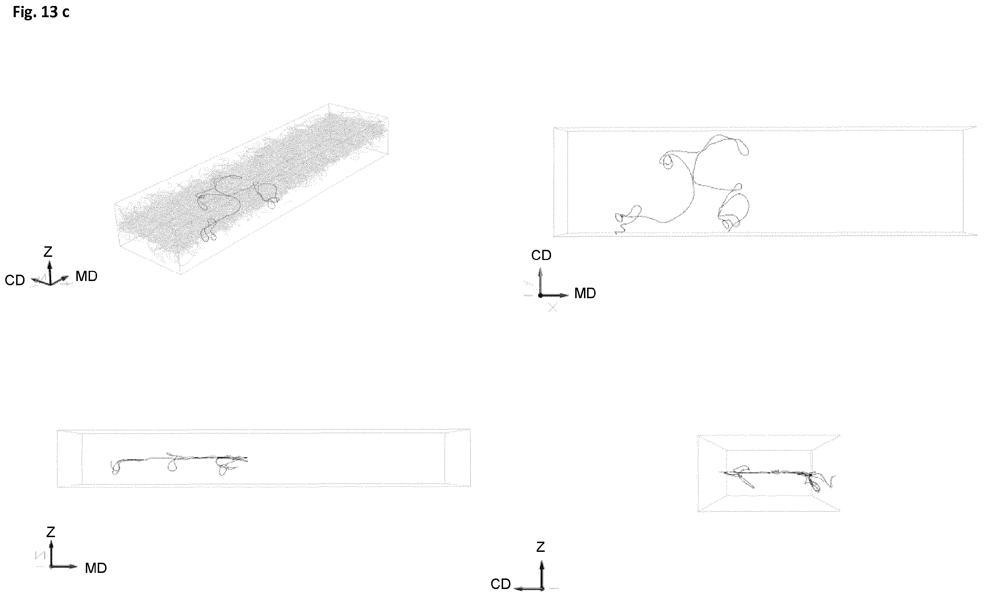

The nonwoven acquisition fabric of the absorbent article is suitably formed from many fibers having many contacts between each other. Looking at it on the millimeter and/or submillimeter scale, we can find that the fibers, or better the millimeter and/or submillimeter parts of fibers are, due to their neighboring fibers, in a unique situation, where they face a unique combination of forces during activation, which results in a huge variety of filament shapes in the final structure. In contrast, the fiber can remain almost perfectly at the planar MD/CD level. On the other hand, the fiber can move "up" or "down" and form a large 3D structure in all MD, CD and z-directions. Some examples can be seen in Fig. 13. Not to be bound by theory we believe that the variety of the filament routes in the layer brings an advantage in the final properties. Preferably at the macroscopic scale, the layer is homogenous. The variety of filament forms comprised within the nonwoven acquisition fabric and their mutual interactions presents the advantage of preferred embodiments, thus the fabric is able to respond to external actions (e.g. pressure and release or fluid going through it) in the desired way. With considerable simplification, we can also express the fiber route by means of the 'length of the filament to the length of the fabric ratio.

Preferably the nonwoven acquisition fabric contains:

at least 20% of fibers with a 'length of the filament to the length of the fabric' ratio greater than 120%, preferably at least 30% of fibers with a 'length of the filament to the length of the fabric' ratio greater than 120%, preferably at least 40% of fibers with a 'length of the filament to the length of the fabric' ratio greater than 120%, more preferably at least 50% of fibers with a 'length of the filament to the length of the fabric' ratio greater than 120%;

at least 10% of fibers with a 'length of the filament to the length of the fabric' ratio greater than 150%, preferably at least 15% of fibers with a 'length of the filament to the length of the fabric' ratio greater than 150%, preferably at least 20% of fibers with a 'length of the filament to the length of the fabric' ratio greater than 150%, preferably at least 25% of fibers with a 'length of the filament to the length of the fabric' ratio greater than 150%, more preferably at least 30% of fibers with a 'length of the filament to the length of the fabric' ratio greater than 150%;

at least 5% of fibers with a 'length of the filament to the length of the fabric' ratio greater than 200%, preferably at least 10% of fibers with a 'length of the filament to the length of the fabric' ratio greater than 200%, preferably at least 15% of fibers with a 'length of the filament to the length of the fabric' ratio greater than 200%, more preferably at least 20% of fibers with a 'length of the filament to the length of the fabric' ratio greater than 200%.

Preferably the nonwoven acquisition fabric contains:

at least 10% of fibers with a 'length of the filament to the length of the fabric' ratio less than 250%, preferably at least 20% of fibers with a 'length of the filament to the length of the fabric' ratio less than 250%, preferably at least 30% of fibers with a 'length of the filament to the length of the fabric' ratio less than 250%, preferably at least 40% of fibers with a 'length of the filament to the length of the fabric' ratio less than 250%, more preferably at least 50% of fibers with a 'length of the filament to the length of the fabric' ratio less than 250%;

at least 5% of fibers with a 'length of the filament to the length of the fabric' ratio less than 200%, preferably at least 10% of fibers with a 'length of the filament to the length of the fabric' ratio less than 200%, preferably at least 15% of fibers with a 'length of the filament to the length of the fabric' ratio less than 200%, more preferably at least 20% of fibers with a 'length of the filament to the length of the fabric' ratio less than 200%.

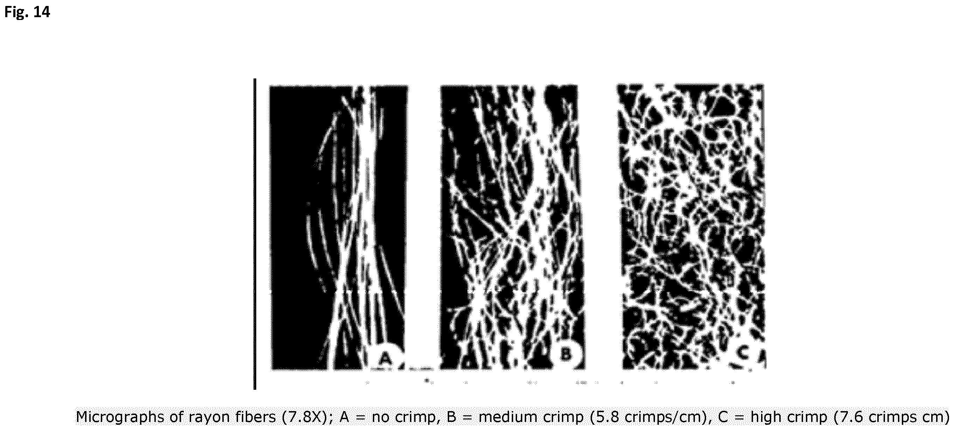

Fibers with a crimpable cross-section tend to form regular shapes - helical crimps, wherein the fibers substantially tend to regularly bend towards that side of the fiber which comprises of the more shrinkable material. Although, they are also limited by their neighboring fibers; the regular force leads them to create substantial helixes. Not to be bound by theory, we believe that the greater the inner shrinkage force, the higher will be the 'crimps per length' unit on single fibers, and, therefore, there will be more helix parts found on the fabric structure. In contrast, when the crimping level is lower, for example, less than 25 crimps per inch (each single "round" on more than 1 mm of formed helix length), the free space between fiber contact points starts to be insufficient for the formation of a proper part of a helix, whilst the opposing forces caused by fiber contacts also become relatively stronger. A person skilled in the art will realize that set crimping numbers are just examples and can differ with various fiber compositions and/or process conditions. Below approximately 15 crimps per inch (each single "round" on more than 2 mm of formed helix length), the parts of helixes are hard to identify and below approximately 10 crimps per inch (each single round on more than approx. 2,5 mm helix length) the regular forces in the fiber are fully overcome by the opposing forces and contrary to the inner shrinkage vector shift and tend towards regular crimp formation, thus the structure may appear to be fully irregular. However, a person skilled in the art will become aware of the different engines driving the bulky structure caused by the regular inner shrinkage vector shift (crimpable cross-section) and the bulky structure caused by irregular fiber shrinkage in the case of a non-crimpable fiber cross-section. Examples of structure differences based on a crimp of rayon fibers can be seen in Fig. 14 (discussed in article Fiber Crimp Distribution in Nonwoven Structure from **Kunal Singha, Mrinal Singha** from 2013 (available at http://article.sapub.org/10.5923.j.fs.20130301.03.html).

Although it is complicated to describe in a general way the structural differences distinguishing a nonwoven fabric created from non-crimpable fibers, and a nonwoven created from crimped fibers, especially in the case of lower crimp levels, a person skilled in the art can confidently determine the type of fabric they are inspecting. For example, there is a comparison of the SEM cross-section images of examples 7C (crimped) and 5A+5D in Fig. 14 - 15.

In case of uncertainty, the component layout in the fiber cross-section becomes the most important factor. The layout may be known from production settings, or it can be estimated using "The type of fiber cross-section estimation" method. Preferably, the nonwoven acquisition fabric of the absorbent article has a thickness (at 0,5 kPa) relative to basis weight (thickness recalculated to 1 g/m$^2$ = thickness (mm) / basis weight (g/m$^2$)) of at least 0,015, preferably of at least 0,020, more preferably of at least 0,025, most preferably at least 0,030.

Preferably, the nonwoven acquisition fabric of the absorbent article has a thickness measured at 0,5 kPa, said thickness is preferably uniform over the entire extension of the nonwoven acquisition fabric.

In a preferred embodiment, the absorbent article comprises a length L1, the nonwoven acquisition fabric comprises a length L2, wherein 0,1 x L1 < L2 < 0,9 x L1. More preferably the nonwoven acquisition fabric is disposed at a distance in the longitudinal direction of the absorbent article from both the back end of the absorbent article and the front end of the absorbent article. Even more preferably, the nonwoven acquisition fabric is disposed at a distance in the longitudinal direction of the absorbent article from both the back end of the absorbent storage core and the front end of the absorbent storage core.

The length L1 of the absorbent article, preferably, is 400 mm - 1200 mm, more preferably 600 mm -1100 mm, more preferably 650 mm -1050 mm, most preferably 700 mm -1000 mm.

In another preferred embodiment the length L1 is 100 mm - 400 mm, preferably 150 mm - 350 mm, in particular if the absorbent article is an incontinence pad.

Preferably, the nonwoven acquisition fabric of the absorbent article has a recovery of at least 0,8 (which corresponds to 80% recovery of the original thickness), preferably of at least 0,82, more preferably of at least 0,84, most preferably of at least 0,85 as defined herein below.

Preferably, the nonwoven acquisition fabric of the absorbent article has the compressibility (as defined herein below) for each 1 g/m$^2$ of layer basis weight of at least 0,25 microns (0,00025 mm), preferably of at least 0,75 microns (0,00075 mm), preferably of at least 1,25 microns (0,00125 mm), more preferably of at least 1,75 microns (0,00175 mm). So, for example, a 100 g/m$^2$ layer has the compressibility of at least 25 microns (0,025 mm), preferably at least 75 microns (0,075 mm), preferably at least 125 microns (0,125 mm), more preferably at least 175 microns (0,175 mm).

Preferably, the nonwoven acquisition fabric of the absorbent article has a resilience as defined herein below of at least 5%, preferably of at least 8%, more preferably of at least 10%, more preferably of at least 13%, more preferably of at least 15 %. Preferably, the nonwoven acquisition fabric of the absorbent article comprises most preferably of filaments with a median fiber diameter of at least 10 microns; preferably of at least 15 microns; more preferably of at least 20 microns. Preferably, the nonwoven acquisition fabric of the absorbent article consist of filaments with median fiber diameter of no more than 50 microns; preferably of no more than 40 microns; more preferably of no more than 35 microns.

The thickness of fibers and also the distribution of fiber thicknesses can affect many other parameters. For example, for certain applications, the homogenous fiber thickness distribution can be taken advantage of, i.e. where the fibers are substantially the same, the vector forces in them are substantially comparable and the final fabric is substantially homogenous. For certain other applications, a wide fiber thickness distribution can be taken advantage of, i.e. where there are thicker and thinner samples within the fabric. Not to be bound by theory, we believe that from a certain level, the vector forces in thick fibers are much stronger than the vector forces in thin fibers and thus thick fibers can become the dominant activator and form the final state of the nonwoven acquisition fabric, whereas the vector force in thin fibers can be suppressed. The final structure, where thick fibers form something like an inner skeleton, can be advantageous, for example, for filtration. The combination of thick and thin fibers can be produced using mixed filaments (mixed spunbond described for example in application WO2009145105A1 from Mitsui) or can be produced using consecutive beams, under the condition that the batt from each beam remains open enough to enable the thick and thin fibers to merge into a single structure.

Preferably, the nonwoven acquisition fabric of the absorbent article has a certain void volume ratio (no unit) when compressed as defined herein below ("compressed void volume ration"), which is defined as the volumetric percentage of the total volume of void spaces in a material with respect to the bulk volume occupied by the material und a certain pressure. A person skilled in the art will know that the void volume can be measured by many different methods. For the purpose of this document, the discussed void volume is calculated from the known basis weight (g/m$^2$), average polymer density of the fibers and known bulk volume (fabric thickness or caliper of 1 m$^2$) at a given pressure.

Preferably, the nonwoven acquisition fabric of the absorbent article has a compressed void volume ratio as defined herein of 0,900-0,990, preferably 0,910-0,985, more preferably 0,920-0,980, more preferably 0,930-0,975, more preferably at least 0,940, in particular at least 0,950.

Even more preferably the nonwoven acquisition fabric has a void volume softness index VVSI as defined herein below of at least 0,50 preferably at least 1,00, more preferably at least 2,00, more preferably at least 3,00, more preferably at least 4,00, more preferably at least 5,00, most preferably at least 6,00.

The nonwoven acquisition fabric of the absorbent article thereby preferably combines another set of key properties, which preferably shall be in proper balance.

Preferably, the nonwoven acquisition fabric of the absorbent article has a MD/CD tensile ratio as defined herein of 0,50-4,00, preferably 0,75-3,00, more preferably 0,80-2,00, in particular 0,85-1,50, in particular 0,90-1,30, most preferably 1,00-1,20.

Preferably, the nonwoven acquisition fabric of the absorbent article is a single, unitary piece.

The absorbent article, preferably, is an incontinence absorbent article, more preferably selected from the group consisting of an incontinence slip, an incontinence pant, and incontinence pad.

More preferably the absorbent article is selected from the group consisting of a T-shape incontinence slip, an H-shape incontinence slip, an H-shape incontinence pant.

The term absorbent article refers to articles which absorb and contain body exudates, and more specifically, refers to devices which are placed against or in proximity to the body of the wearer to absorb and/or contain the various body fluids or exudates discharged from the body.

Herein, the term incontinence absorbent article refers to absorbent articles which are designed to be preferably used by incontinent persons, typically adult incontinent persons.

The term incontinence slip refers to articles that can be assembled on a wearer by securing fasteners when the garment is in place on a wearer, typically allowing to accomodate the waistregion of the article to fit a specific hip size of the user by varying degree of overlapping of the front parts with the rear parts of the article.

A T-shape incontinence slip refers to incontinence slips having a width of the rear section greater then a width of the front section of the article. Preferably T-shape incontinence slips are configured as shown in WO2017114695A1. In particular T shape incontinence slips consist of a central chassis including an absorbent component and rear side panels disposed at both side edges of the chassis in the rear section while the article typically has no front side panels. Typically said pair of back side panels are separately attached to said central chassis as shown e.g. in WO2017114695A1. Preferably the maximum span MS of the T-shape incontinence slip provides for a ratio of the maximum span MS of the incontinence slip 200 to a front span VS of the incontinence slip of preferably 1,3 - 2,8, in particular 1,4 - 2,7, further in particular 1,5 - 2,6, further in particular 1,6 - 2,5.

An H-type incontinence slip refers to incontinence slips having both a pair of front side panels and a pair of rear side panels, typically separately attached to respective front and rear sections of a central chassis, said chassis comprising an absorbent component. Preferably, H-shape incontinence slips are configured as shown in EP1763329A1 or EP2410965A1.

The term incontinence pant refers to articles which are typically three-dimensional products with closed sides so that the product has a unitary waist opening and two leg openings and which are pulled onto the body of the wearer by inserting the legs into the leg openings and pulling the article up over the waist (EP1906901A1). Incontinence pants may, however, be configured to be refastenable as shown in WO2004016207A2.

The term H-shape incontinence pant refers to incontinence pants having a front body panel comprising a terminal waist edge and a terminal crotch end and a rear body panel comprising a terminal waist edge and a terminal crotch edge, wherein said terminal crotch edge of said rear body panel is longitudinally spaced from and forms a gap with said terminal crotch edge of said front body panel, and wherein an absorbent insert bridges said gap and is connected to said front and rear body panels (e.g. as disclosed in EP1572057A1 or EP2849706A1).

The term incontinence pad (or incontinence insert) refers to absorbent articles that are placed against or in proximity to the body of the wearer to absorb and contain various exudates discharged from the body and are not configured as slips or pants. Such articles are frequently offered to consumers in individual wrappers or envelopes prior to use, in order to preserve their cleanliness until actual use. During use, such articles are often held in place to an undergarment via either another article which holds the pad such as an elastic net pant or via one or more pressure sensitive adhesive patches or strips, or alternatively, hook and loop style fasteners, positioned on the garment-facing surface of the backsheet layer. Some of these articles also include wing-like structures or extending foldable tabs, for wrapping about the edges of a user's undergarments to further secure them. Such wing-like structures are frequently integral with the absorbent article body, and are constructed from discrete, lateral extensions of both the topsheet and backsheet layers. Alternatively, the wing-like structures may be formed as separate attachments to the article.

The absorbent article, preferably, has an ISO absorption capacity (as defined herein below) of less than 300 g. Absorbent articles of less than 300 g ISO absorption preferably serve for cases of light incontinence.

In another preferred embodiment, the absorbent article has an ISO absorption capacity (as defined herein below) of equal to or more than 300 g more preferably more than 500 g, more preferably more than 700 g, more preferably more than 900 g, even more preferably more than 1100 g. Absorbent articles of this kind preferably serve for cases of medium and severe incontinence.

Preferably the absorbent article comprises a topsheet and a backsheet, and more preferably the absorbent storage core is disposed between the nonwoven acquisition fabric and the backsheet.

Suitable known materials for the absorbent storage core include fiber material, preferably cellulose fibers, preferably in the form of comminuted wood pulp, commonly known as "airfelt", natural or synthetic fibrous materials, and superabsorbent polymers, used either singly or in mixtures and commonly formed into layers or sheets, etc.

The term "superabsorbent" refers herein to a water-swellable, water-insoluble organic or inorganic material capable, under the most favorable conditions, of absorbing at least about 15 times its weight and, in an embodiment, at least about 30 times its weight, in an aqueous solution containing 0.9 weight percent sodium chloride. The superabsorbent materials can be natural, synthetic and modified natural polymers and materials. In addition, the superabsorbent materials can be inorganic materials, such as silica gels, or organic compounds, such as cross-linked polymers.

Preferably, the absorbent storage core comprises less than 90%, more preferably less than 80%, more preferably less than 70%, more preferably less than 60%, more preferably less than 50%, more preferably at least 10%, more preferably at least 20%, more preferably at least 30%, most preferably at least 40% by weight superabsorbent material (SAP). In this context % means weight-%.

In an alternative embodiment the absorbent storage core comprises components or layers comprising more than 50%, more preferably more than 60%, more preferably more than 70%, more preferably more than 80%, more preferably more than 90%, (by weight) superabsorbent material.

Preferably, the absorbent storage core comprises components free of cellulose fiber material.

In another preferred embodiment, at least a component (e.g. a layer) of the absorbent storage core has a first area and a second area, the basis weight of the absorbent material of the first area is different from the basis weight of the absorbent material of the second area.

Even more preferred, the second area is free of absorbent material. Even more preferred, the nonwoven acquisition fabric covers at least in part, preferably entirely the second area.

Preferably the second area comprises at least one channel, more preferably one single channel. Preferably, the channel is disposed at least in part, more preferably all the way along the longitudinal centerline of the absorbent article.

By "channel", it is meant that the structure or layer referred to comprises an absorbent material free area, forming a visible conduit or passage typically extending along the length direction of the core. A channel can take a variety of forms, especially rectangular, hourglass, oval or lenticular. A channel, preferably, has a maximum longitudinal extension and a maximum transverse extension, whereby the maximum longitudinal extension is greater than the maximum transverse extension.

In another preferred embodiment, at least a component (e.g. a layer) of the absorbent storage core has a first area and a second area, the thickness (at 0,5 kPa) of the absorbent material of the first area is different from the thickness (at 0,5 kPa) of the absorbent material of the second area.

Even more preferred, the second area is an embossed area. Even more preferred, the nonwoven acquisition fabric covers at least in part, preferably entirely the second area. Preferably the second area comprises at least one embossed area, more preferably 2-5, in particular 2-3 embossed areas. Preferably the embossed areas are groove-like embossed areas, meaning the embossed area has a maximum longitudinal extension and a maximum transverse extension, whereby the maximum longitudinal extension is greater than the maximum transverse extension.

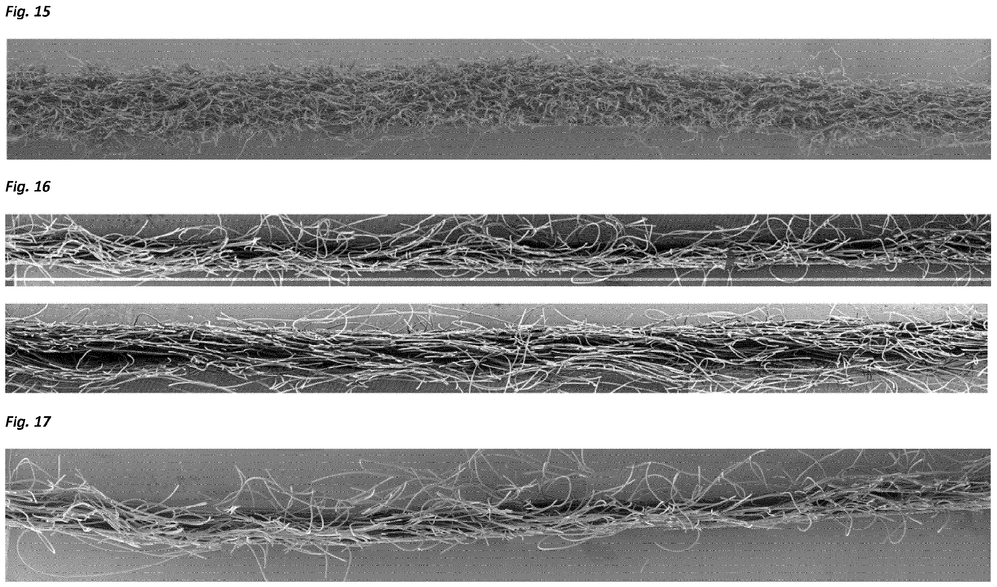

In another preferred embodiment, the nonwoven acquisition fabric is provided with a multiplicity of singular filament loops and/or loop bundles protruding from the surface to the outside, as illustrated on the microscope image (Fig. 17). Not to be bound by a theory, we believe that these "hairs" on the surface have at least two functions:

a. For example these hairs help to interconnect the nonwoven acquisition fabric structure with the topsheet (if present) on the one side and on the other side with the absorbent storage core below the nonwoven acquisition fabric. This interconnection of fibrous structures of the layers in an absorbent article improves the fluid transfer through the layers into the absorbent storage core.

b. For example, in an application requiring direct contact with the user's skin, these hairs improve the tactile softness and make the fabric more pleasant to touch and/or wear.

Preferably, the topsheet of the absorbent article is a separate element, different from the acquisition component. In this case the acquisition component including or consisting of the nonwoven acquisition fabric is disposed between the topsheet and the absorbent storage core.

Advantageously, the nonwoven acquisition fabric of the absorbent article consists of one layer as defined herein, i.e. the filaments the nonwoven acquisition fabric consists of are the same.

In another embodiment the nonwoven acquisition fabric has or consists of more than one layer, preferably 1-4 layers, more preferably two layers. In the latter case it has or consists of an upper layer and a lower layer, wherein the upper layer is preferably making up at least part of the body facing side of the absorbent article, in particular by the upper layer is constituting the topsheet, and wherein the lower layer is facing, preferably directly facing, the absorbent storage core.

Preferably, in this embodiment the topsheet is integral with the acquisition component, in particular integral with the nonwoven acquisition fabric. These two layers in that nonwoven acquisition fabric can be produced as a multi-layered material in one process.

The nonwoven acquisition fabric of the absorbent article of the invention is suitably produced by a method for producing a nonwoven web from continuous filaments, in particular from continuous filaments of thermoplastic material. In the context of the invention nonwoven fabric layers comprising or consisting of continuous filaments are used. It is known that due to their quasi-endless lengths, continuous filaments differ substantially from staple fibers, which have much shorter lengths, for example 10 mm to 60 mm.

The method for producing the nonwoven acquisition fabric is characterized by at least one nonwoven layer being formed as a spunbonded nonwoven acquisition fabric by means of a spunbond process. Preferably, the multicomponent or bicomponent filaments of the nonwoven acquisition fabric layer are spun by a spinning device or spinneret and then passed preferably for cooling through a cooling device. In the cooling device, the filaments are conveniently cooled using a fluid medium, in particular by means of cooling air. Preferably the spun filaments are then passed through a drawing device, and the filaments are drawn. The drawn filaments are then deposited on a tray - preferably laid on a formation moving belt to form a nonwoven batt. In particular, by adjusting the parameters which are controlling the draw down ratio, filaments with a controlled shrink potential can be created within the nonwoven layer. Preferably, a diffuser interposed as a storage device managing the laying down of the filaments is installed between the drawing device and the deposition location. Preferably, at least one diffuser, having divergent opposite side walls in relation to the flow direction of the filaments is utilized. Preferably the drive unit of the cooling device and the drawing device are designed as a closed system. In this closed system, in addition to the supply of the cooling medium or cooling air into the cooling device, no further air supply from outside is utilized. Such a closed system has proven itself superior in the production of nonwovens.

It has been found that the shrinkage is particularly reliable in operation and effectively released when the closed unit described is used and when it is at least used in addition to a particularly preferred embodiment, a diffuser between the drawing device and storage. It has already been indicated that the shrinkage potential of the nonwoven sheet, produced by means of the spunbond method, can be adjusted or controlled very specifically by the parameters draw down ratio, cooling air / polymer ratio and filament speed.

As already defined, the spunbond production method is the direct conversion of polymers into continuous filaments, which are subsequently laid in a deposition location in a random fashion to create a nonwoven layer comprised of these filaments. The spunbond process defines both the properties of a single filament as well as the properties of the final nonwoven acquisition fabric. It is not always possible to use the finished nonwoven acquisition fabric to determine the various properties and states of individual filaments, such as rheological properties, polymer structural properties and shrinkage potential, present during the individual nonwoven production process steps. In general, the shrinkage potential of a nonwoven layer determines its ability to generate a bulky nonwoven by utilizing the shrinkage of single filaments into an increased relative thickness of the filament batt, however, without disintegrating the fabric structure and/or changing the length and the width of the filament batt significantly.

The capacity for fibers to shrink is defined by utilizing different raw materials in the composition of continuous filaments and/or by setting different process conditions in the production of the continuous filaments of the nonwoven acquisition fabric and/or by utilizing different filament cross-section shapes in continuous filaments and/or adjusting the mass ratio between the different input materials and/or by arranging different orientations of the continuous filaments.

A method is characterized by a nonwoven acquisition fabric that is produced from multicomponent filaments, in particular bicomponent filaments, having substantially non crimpable cross-sections, in a core-sheath configuration or other bicomponent fibers with a substantially noncrimpable configuration (Fig. 9). The multicomponent or bicomponent configuration should not be able to generate internal forces within the filament which can initiate regular crimping or curling of the filament.

The first component of the filament, forming, for example, the core, is comprised of polymeric material A that is able to shrink under certain conditions. The second component of the filament, forming, for example, the sheath, is comprised of polymeric material B, which differs from polymeric material A. For example, it contains a different polymer or blend of polymers. Advantageously, the difference between the melting temperature of polymeric material A and the melting temperature of polymeric material B, according to a preferred embodiment of the invention, is greater than 5 °C, preferably greater than 10 °C. The first component can be chosen from a group of polyesters (e.g. from aromatic polyesters such as polyethylene terephthalate (PET), or from aliphatic polyesters such as polylactic acid (PLA)), polyamides, polyurethanes or their copolymers or suitable blends. Preferably, the first component consists or consists essentially of a plastic of the group of polyesters that also includes polyester copolymers (coPET) or polylactide copolymers (COPLA). Preferably polyethylene terephthalate (PET) or polylactic acid (PLA) is used as the polyester.

The second component can be chosen from a group of polyolefins (i.e. polypropylene or polyethylene), low-melting polymers, copolymers or blends of suitable polymers. Preferably, the second component consists or consists essentially of a plastic of the group of polyesters that also includes polyester copolymers (coPET) or polylactide copolymers (COPLA). Preferably polyethylene (PE) is used as the polyolefin. The preferred combination of components for the bicomponent filaments in the nonwoven acquisition fabric according to the invention are PET / PE, PET / PP, PET / CoPET, PLA / COPLA, PLA / PE and PLA/ PP.

The preferred bicomponent filaments have the ratio of the mass of the first component to the mass of the second component from 50:50 to 90:10. It is in the context of the process that the mass ratios of the core/sheath configuration can be freely varied during production without stopping the machine.

A person skilled in the art will appreciate the process advantages provided by filaments with noncrimpable cross-sections over crimping filaments in achieving bulky and soft-loft materials. Unlike non-crimpable fibers, filaments that exhibit (self)crimping during production are not easy to control. Most of the crimpable cross-section filament types develop crimps during the laydown process and/or with activation. Since they move relative to each other during the crimping process, they can easily touch each other or entangle, in different words they can hinder each other. Thus, nonwoven layers consisting of self- crimping filaments are often limited in their design due to the uneven fiber distribution caused by the relative movement of the filaments. The resulting necessary workarounds often include reduced throughput, slower production speeds and special intermediate process steps for fixing the filaments to each other.

Preferably, the nonwoven acquisition fabric of the absorbent article does include less than 20% (by weight), preferably less than 15%, more preferably less than 10%, more preferably less than 5%, more preferably less than 2% filaments having a crimpable cross-section, most preferably the nonwoven acquisition fabric does not include any filaments having a crimpable cross-section.

In a preferred embodiment, all components, i.e. all polymer components of the filaments are arranged across the cross-section of the filament in a non-crimpable configuration.

The nonwoven acquisition fabric of the absorbent article, preferably does not utilize self-crimping filaments and thus a far more uniform laydown can be achieved, which enables a lower possible basis weight while retaining requested fabric properties and/or higher production line speeds with higher throughputs. With non-crimping filaments, the production process is much easier to control and the production of spinning nozzles / spinning beams is cheaper. Therefore, the nonwoven acquisition fabric is less expensive compared to other nonwoven webs, produced by different technologies.

Preferably the resulting nonwoven layer is thermally pre-bonded, i.e. pre-consolidated, thermally activated and thermally bonded. Thermal activation and bonding is preferably carried out with the aid of at least one hat fluid and/or by contact with a hat surface. The hat surface may be part of a roller in particular.

It is desired that thermal activation is performed under the condition that the shrinkage occurs uniformly over the entire surface of the fibrous layer. Thermal activation can be performed in a hot-air box or the batt can be passed through an oven. Thermal activation and bonding can also be performed by means of UV light, carried microwave and/or laser irradiation. It should be emphasized that thermal bonding in the context of the present "in-line" process can also be carried out immediately following the completion of upstream process steps or both the process steps of thermal activation and bonding may be "offline", thus decoupled from the upstream process steps. Thermal activation can, therefore, in principle, be performed "offline" at another time and in another place. Thus, a nonwoven that is not yet thermally activated and still not very bulky can be transported in a simple and space-saving manner to another processing location.

The desired level of pre-consolidation of the web/batt is highly dependent on the production process conditions. The key is to correctly set the level of fiber-to-fiber cohesion within the batt and, thereby, control the level of batt coherence based on the requirements of the subsequent production step. In the case of an inline production process with activation on the belt itself, the desired level of cohesion is rather low, and required only for preventing tears or thinning caused by significant undesirable fiber movements during the activation process. In special cases, for example when the fibers themselves provide very good cohesion in contact with each other or their underlay, caused, for example, by their cross-section shape, entanglement rate or material composition, the cohesion of the batt may be good enough even without thermal pre-consolidation. In other cases, for example when the production process is divided into two steps and when prior to full activation the pre-consolidated batt is transported for example in the form of rolls, the required level of cohesion is much higher and so the pre-consolidation level also needs to be far higher. Persons skilled in the art having knowledge of their process conditions will easily recognize the level of pre-consolidation required for their specific case. The activation temperature shall be in the interval range between glass transition temperature and softening temperature (vicat softening temperature ISO DEN 306) of component A, preferably the core component. A person skilled in the art will recognize the optimal activation temperature for the given composition of the component.

The defined method for producing the nonwoven acquisition fabric of the absorbent article provides bulky nonwoven acquisition fabrics formed using filaments with adjusted or controlled shrinkage potential of the nonwoven filaments. The shrinkage occurs uniformly over the entire batt, thus the process should provide uniform nonwoven properties ensuring uniform controlled shrinkage.

In the cooling device, the filaments are conveniently cooled by a fluid medium, in particular by cooling air. As already mentioned the potential shrinkage of the filaments needs to be uniformly distributed across the full length, width and thickness of the final nonwoven. Shrinkage characteristics can be modified by adjusting draw down ratio, cooling air / polymer ratio and filament speed, thus these parameters are almost uniform for each individual filament.

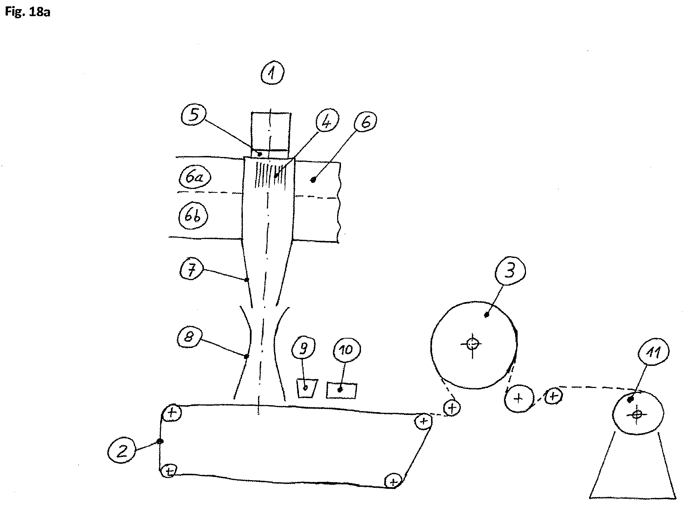

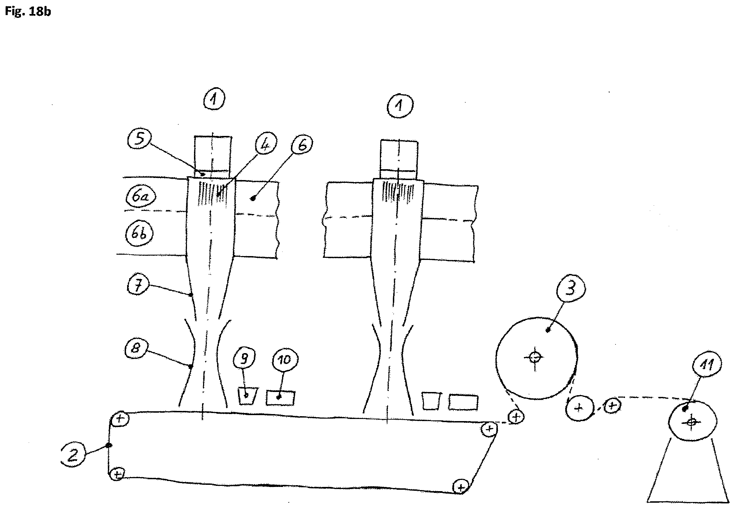

The formed nonwoven acquisition fabric may consist of several layers, each formed on a spunbond beam 1 (See Figure 18A and 18B). It is understood that multiple layers are laid on top of each other and transported together on at least one forming belt 2 to a final bonding device 3. The filaments 4 are spun from a spinneret 5. The arrangement of the filaments is optimized by a staggered arrangement so that each filament gets a very similar mass and a very similar temperature of cooling air. The spinnerets can vary in number of capillaries as well as in the diameter (d) and the length (l) of the capillaries. The length l is typically calculated as multiple of the capillary diameter and for this application is in the range from 2 to 10 l/d. The number of capillaries must be chosen based on the required final filament diameter and the required or planned total polymer throughput together with the required filament spinning speed. The number of capillaries can be varied from 800 - 7000 capillaries per meter, providing a filament diameter range from 5 to 50 μm. The capillary diameter and the filament speed are chosen in order to be able to generate the right level of shrinkage potential in the final filament. Filament speed should be defined between 3000 and 5500 m/min, the capillary diameter should be in between 200 and 1000 μm, resulting in a draw down ratio suitable for the process from 200 to 1300 in case of round capillaries, to reach the desired level of line productivity, more suitable for the process is a draw down ratio from 300 to 800 in case of round capillaries. Non-round capillaries show typically higher draw down ratios, greatly dependent on the capillary shape and its surface-to-volume ratio. The volume and temperature of the cooling air is set to achieve the correct draw down ratio and cooling conditions. Helpful for this, a cooling air / polymer ratio of 20 to 45 has been identified. The volume and temperature of the cooling air is controlled in the cooling device 6. The temperature can be set between 10°C and 90°C, preferably the temperature can be set between 15°C and 80°C so that the shrinkage can be controlled by the cooling conditions. The cooling conditions define how fast the filaments cool down from melt temperature at spinning to glass transition temperature. For example, a higher cooling air temperature results in a delayed cooling of the filaments. In order to achieve the required and useful temperature range for the cooling air, in practice it is easier to handle temperature ranges when the cooling device is divided into 2 different zones in which the temperature can be controlled separately. In the first zone (6a), which is near to the spinneret, the temperature can be set between 10°C and 90°C, preferably the temperature can be set between 15°C and 80°C and most preferably between 15°C and 70°C. In the second zone (6b), which is close to the first zone, the temperature can be set between 10°C and 80°C, preferably the temperature can be set between 15°C and 70°C and most preferably between 15°C and 45°C.

Thereafter, the filaments are guided through the draw down zone 7. The filaments are drawn down by pulling forces created by the air speed of the cooling air. The volume of cooling air and the adjustable geometry of the draw down zone results in an air speed, which is also converted into filament speed. The filament speed together with the polymer throughput also defines the filament diameter. Potential shrinkage is controlled by the filament speed, the draw down ratio and the cooling air / polymer ratio.

In the next step, the filaments are guided to the diffuser 8 which has divergent side walls in relation to the flow direction of the filaments. These walls can be adjusted and are adjusted in a way to achieve a uniform nonwoven acquisition fabric in which single filaments create a filament laydown arrangement exhibiting omnidirectional orientation in the MD/CD plane.

It is understood that a filament laydown is influenced by the air guiding the filaments in the diffuser. The air can be adjusted to create arrangements from distinct zigzag lay down arrangements to real round loops, and furthermore CD-orientated elliptical structures. The filaments are laid down on the formation belt and transported into at least one pre-consolidation device 9. Cooling air is moved through the filament lay down layer and the formation belt out of the process. The volume of suction air can be adjusted to help the filament lay down and also to ensure that the filament batt is fixed on the formation belt. The pre-consolidation device is located close to the diffuser. The filament batt is controlled on the way from the diffuser to the pre-consolidation device by suction air. The pre-consolidation of the filament batt is performed by means of hot air.

The energy transferred onto the filament batt is controlled in a way that the filaments are only partly softened or pre-melted to generate good cohesion between individual filaments. Having achieved good filament cohesion, the filament batt can be transported on the formation belt without further help from any other device and without being influenced or destroyed/damaged by the transportation forces. This pre-consolidation process is also sufficient enough to run the filament batt into another lay down zone on a multi beam production line. The energy transferred to the filaments is not sufficient to activate the shrinkage of the filaments.

The method describes the balance of the pre-consolidation parameter, pre-consolidation temperature, pre-consolidation air speed and pre-consolidation time. Pre-consolidation time is understood to be the time during which the filament batt is treated by the pre-consolidation air.

Pre-consolidation time for the batt is recommended to be between 1 and 10000 ms, preferably between 2 and 1000 ms and most preferably between 4 and 200 ms. Pre-consolidation air speed used in this pre-consolidation device is adjustable between 0,1 and 10 m/s, preferably in between 0,8 and 4 m/s. It is recommended that the pre-consolidation temperature of the pre-consolidation is between 80°C and 200°C, preferably between 100°C and 180°C. In one embodiment, the pre-consolidation temperature is 90°C to 150°C, in particular 110°C to 140°C.

According to a preferred embodiment, the bicomponent filaments of the nonwoven acquisition fabric of the absorbent article have a core component made of polyethylene terephthalate (PET) and a sheath component of a polyolefin particularly polyethylene or polypropylene, preferably where the pre-consolidation temperature is preferably 110°C to 130°C, and in particular 120°C to 150°C.

In one embodiment, the nonwoven fabric comprises or consist of bicomponent filaments, the core component made of polyethylene terephthalate (PET) and the sheath component of polyethylene terephthalate copolymer (CoPET), for which the pre-consolidation temperature is preferably 110°C to 180°C.

When the nonwoven layer is comprised of bicomponent filaments having a core component made of polylactic acid (PLA) and a sheath component of a polyolefin, in particular from a polyethylene or polypropylene, the pre-consolidation temperature is preferably 80°C to 130°C.

Further down the production line from the diffuser, the filament batt is transported into at least 1 activation unit 10 (Figures 18A, 18B). The filaments are activated by means of hot air. It is understood that the actual shrinkage of the shrinkable component of the filament is a function of the temperature of the shrinkable component of the filament and also the duration of the temperature exposition. It is also well understood that the speed of the shrinkage process depends on the temperature of the shrinkable component of the filament. The process should be controlled in a way that the shrinkage is introduced slowly, so the forces introduced into the filament batt from the shrinkage are lower than the cohesion forces between the filaments. The result of this process control is a cohesive and uniform nonwoven acquisition fabric structure with a reduced filament structure density, which also results in an increased thickness of the nonwoven.

One embodiment of the method is to join the process steps of pre-consolidation and activation by controlling the pre-consolidation and/or activation time, the pre-consolidation and/or activation air speed and the pre-consolidation and/or activation temperature in a combined pre-consolidation and activation device.

The innovative method describes the balance of the activation parameters: activation temperature, activation air speed and activation time. The activation time is understood as the time during which the filament batt is treated by the activation air. It is well understood that these parameters can be varied in the mentioned ranges in order to react to the potential shrinkage level in the filaments as well as to set the ideal combination between activation time, activation temperature and activation air speed.

The activation time for the batt is recommended between 20 and 5000 ms, preferably between 30 and 3000 ms and most preferably between 50 and 1000 ms. The activation air speed used in this activation unit is adjustable between 0,1 and 2,5 m/s, preferably between 0,3 and 15 m/s. It is recommended that the activation temperature of the thermal activation is between 80°C and 200°C, preferably between 100°C and 130°C. In one embodiment, the activation temperature is 90°C to 140°C, in particular 110°C to 130°C. According to a preferred embodiment, the nonwoven layer of bicomponent filaments has a core component made of polyethylene terephthalate (PET) and a sheath component of a polyolefin particularly polyethylene or polypropylene, the activation temperature is preferably 90°C to 140°C and in particular 100°C to 140°C. In one embodiment, the nonwoven layer comprises of bicomponent filaments, the core component is made of polyethylene terephthalate (PET) and the sheath component of polyethylene terephthalate copolymer (CoPET), the activation temperature is preferably 120°C to 160°C. When the nonwoven layer comprises of bicomponent filaments having a core component made of polylactic acid (PLA) and a sheath component made of a polyolefin in particular polyethylene or polypropylene, the activation temperature is preferably 80°C to 140° C.

A method for arriving at the nonwoven acquisition fabric of the acquisition component of the absorbent article prescribes a final bonding procedure of treating the filament batt with hot air in a bonding device. The nonwoven acquisition fabric of the absorbent article of the invention therefore is an air-through bonded nonwoven acquisition fabric. In the bonding device the filament batt of a single layer or more layers is bonded together without reducing the thickness of the filament batt significantly and having almost no bonding gradient throughout the thickness of the nonwoven. It is well understood that the remaining thickness and the resilience of the nonwoven is influenced by the bonding temperature since the bonding temperature should be high enough to achieve the needed bonds between the nonwoven fibers, without softening and collapsing the filament batt. In the bonding device, the bonding temperature and forces applied to the filament batt need to be adapted to the required process effect of low softening and low forces but sufficient to affect the integrity of the nonwoven filament batt. This can be achieved in multiple different devices like an omega drum bonding device, a flat belt bonding device as well as a multiple drum bonder.

The bonded nonwoven is finally wound up on a winder 11 (Figures 18A, 18B). In case surface properties of the nonwoven have to be modified for example to achieve improved fluid transportation or wicking performance a spraying device or kiss roll is placed either in between the forming belt and the final bonding device or in between the final bonding device and the winder.

One embodiment is to connect the process steps of activation and bonding by controlling the activation and/or bonding time, activation and/or bonding air speed and activation and/or bonding temperature in the bonding device.

The method describes the balance of the bonding parameter bonding temperature, bonding air speed and bonding time. The bonding time is understood to be the time during which the filament batt is treated with the bonding air. It is well understood that this parameters can be varied in the mentioned ranges in order to react to the bonding potential of the filament batt as well as to achieve the ideal combination in between bonding time, bonding temperature and bonding air speed. The bonding time for the batt is recommended between 200 and 20000 ms, preferably between 200 and 15000 ms and most preferably between 200 and 10000 ms.

The bonding air speed used in this bonding unit device is adjustable between 0,2 and 4,0 m/s, preferably between 0,4 and 1,8 m/s. It is recommended that the bonding temperature for thermal bonding is between 100°C and 250°C, preferably between 120°C and 220°C. In one embodiment, the bonding temperature is 90°C to 140°C, in particular 110°C to 130°C. According to a preferred embodiment, the nonwoven layer of bicomponent filaments has a core component made of polyethylene terephthalate (PET) and a sheath component from a polyolefin, particularly polyethylene or polypropylene; the bonding temperature is preferably 90°C to 140°C and in particular 100°C to 140°C. In one embodiment, the nonwoven layer is comprised of bicomponent filaments, the core component is made from polyethylene terephthalate (PET) and the sheath component is made from polyethylene terephthalate copolymer (CoPET), the bonding temperature is preferably 140°C to 230°C. When the nonwoven layer is comprised of bicomponent filaments having a core component made from polylactic acid (PLA) and a sheath component made from a polyolefin, in particular from a polyethylene or polypropylene, the bonding temperature is preferably 80°C to 140°C. The mentioned temperature ranges can be used in different discrete steps, so that the bonding air temperature as well as the bonding air speed remain within the mentioned range but in different zones of the bonding device at different levels.

The nonwoven acquisition fabric of the absorbent article can on the one hand be designed to be relatively bulky and thus exhibit a relatively large thickness, whilst on the other hand, nevertheless, retain satisfactory stability. The nonwoven acquisition fabrics have an excellent resilience after being subjected to a load or a pressure load. These advantageous properties can be achieved at relatively low basis weights of the nonwoven acquisition fabric.

The method is further characterized by the advantage that, a continuous production of the nonwoven acquisition fabric at relatively high production speeds without interruption of the production process is possible in a simple manner. The parameters for the production of the nonwoven acquisition fabric are highly variable and flexible, adjustable during the process and therefore variable end products can be produced without interrupting the production process. Also, pre-consolidation, activation and bonding steps can be easily varied with respect to the parameters.

The method can be performed in a simple way "inline", whilst still being able to be easily performed "offline" if necessary. Thus, the pre-consolidation, activation of shrinkage, and the final bonding can be uncoupled without any problems from actual laminate production. In summary, it should be noted that an innovative fabric having a very advantageous 3D structured surface with high volume and large thickness can be produced with satisfactory compression strength of the fabric in a simple, inexpensive and cost-effective manner. Various parameters of the nonwoven acquisition fabric or of the resulting nonwoven layer are variable and flexibly adjustable during the production process.

In a further embodiment which is, however, not part of the invention, an array is hereby proposed, said array comprising a first type of absorbent article and a second type of absorbent article different from the first type of absorbent article, wherein both types of absorbent articles comprise an absorbent storage core and an acquisition component, the acquisition component comprises a nonwoven acquisition fabric, the nonwoven acquisition fabric comprises filaments, the filaments comprise a first polymeric material and a second polymeric material, the second polymeric material having its melting point lower than the first polymeric material, wherein the second polymeric material extends in the longitudinal direction of the filaments and forms at least a part of the surface of the filaments, and the nonwoven fabric comprises filament-to-filament bonds formed of the second polymeric material wherein the nonwoven acquisition fabric has a structural softness as defined herein of at least 80 $m^4mm^2g^{-2}$ and wherein the first and the second type of absorbent articles are selected from the group consisting of a T-shape incontinence slip, an inverted H-shape incontinence slip, an H-shape incontinence pant, an incontinence pad.

More preferably a series of properties of the nonwoven acquisition fabric of the first disposable article of said array are equal to the properties of the nonwoven acquisition fabric of the second disposable article, wherein the series of properties is at least two or three or four or five or six or seven or all of the group consisting of basis weight, density, fibers constituting the nonwoven acquisition fabric, compressibility, shape, width, length, area, structural softness, compressed absolute void volume, void volume softness index VVSI.

Preferably, the nonwoven acquisition fabric of the first and second absorbent article can be configured to any of the above embodiments, forms, compositions and properties.

Preferably, the nonwoven acquisition fabric has a compressed absolute void volume as defined herein of at least 0,010 $m^3$/kg, preferably at least 0,013 $m^3$/kg, more preferably at least 0,016 $m^3$/kg, more preferably at least 0,019 $m^3$/kg, more preferably at least 0,022 $m^3$/kg, more preferably at least 0,025 $m^3$/kg, most preferably at least 0,028 $m^3$/kg.

In a further embodiment which is, however, not part of the invention, an other array is hereby proposed, said array comprising a first and a second absorbent article of the same type wherein the first and second absorbent article comprise an absorbent storage core and an acquisition component, the acquisition component comprises a nonwoven acquisition fabric, the nonwoven acquisition fabric comprises filaments, the filaments comprise a first polymeric material and a second polymeric material, the second polymeric material having its melting point lower than the first polymeric material, wherein the second polymeric material extends in the longitudinal direction of the filaments and forms at least a part of the surface of the filaments, and the nonwoven fabric comprises filament-to-filament bonds formed of the second polymeric material wherein the nonwoven acquisition fabric has a structural softness as defined herein of at least 80 $m^4mm^2g^{-2}$ and wherein the same type of absorbent article is selected from the group consisting of T-shape incontinence slip, an H-shape incontinence slip, an H-shape incontinence pant, an incontinence pad, wherein the first absorbent article has a first size and the second absorbent article has a second size different from the first size.

The size of the articles typically affects, for example, the size of the waist opening, the size of the openings around the thighs (both in the case of a pant), or the length or the width of the absorbent article. Preferably, the nonwoven acquisition fabric of the first and second absorbent article of the same type can be configured to any of the above embodiments, forms, compositions and properties.

Preferably a series of properties of the nonwoven acquisition fabric of the first disposable article of the first size is equal to the properties of the nonwoven acquisition fabric of the second disposable article of the second size, wherein the series of properties is at least two or three or four or five or six or seven or all of the group consisting of basis weight, density, fibers constituting the nonwoven acquisition fabric, compressibility, shape, width, length, area, structural softness, compressed absolute void volume, void volume softness index VVSI.

Preferably, the nonwoven acquisition fabric of the first and second absorbent article of the same type can be configured to any of the above embodiments, forms, compositions and properties.

An array as referred to herein results from the obvious preparation of the absorbent articles belonging to the array. In particular by the relation of the articles to each other. This is done either by presentation in a common packaging unit and/or preferably by the application of markings on the absorbent articles and/or their packaging and/or the presentation in spatial or content-related proximity to each other, which indicate the affiliation to an array. The absorbent articles forming the array preferably originate from one and the same manufacturer. The absorbent articles forming an array preferably have the same product identification as brand names and/or sub-brand names.

Examples

A nonwoven acquisition fabric of the absorbent article of the invention can be produced, for example, on a laboratory line at UTB Zlfn Centre of Polymer Systems Czech Republic. The laboratory line model LBS-300 is able to produce spunbond or meltblown fibers in mono or bicomponent compositions. Its extrusion system, consisting of two extruders is able to heat polymers up to 450°C. Spunbond fibers can be produced using a spunbond die containing 72 holes (0,35 mm diameter; 1,4 mm length) on a square area of 6x6 cm. There are several possible bicomponent die configurations - core/sheath, side/side, segmented pie or islands-in-the-sea. The system is open; stretching air pressure in the inlet system is available up to 150 kPa. Filaments can be collected as they are or can be laid down on a belt at speeds from 0,7 to 12 m/min. The final product width is up to 10 cm. The total throughput rate can be set from 0,02 to 2,70 kg/h. The final basis weight of the product can be set between 30 and 150 g/m². There is an option to bond the batt using a calender roll at a temperature of up to 250 °C. This laboratory line was used to produce layers described in examples 1-4. To model air-through-bonding at the laboratory (examples 1-4), a standard stationary oven was used. Due to the very different heat transfer conditions present at the oven with a static atmosphere and the air stream forced to go through the fabric, and due to the heat loss during the opening and closing the oven, the activation time was set to 5 minutes.

Example 1

The nonwoven acquisition fabric consists of bicomponent filaments with a noncrimpable cross-section layout, core/sheath type, where the core/sheath mass ratio is 70:30, the core is formed using PLA (Ingeo from Nature Werks) and the sheath is formed using PP (Tatren HT 2511 from Slovnaft). The nonwoven was produced on a laboratory line at UTB Zlfn, Centre of Polymer Systems. The core extruder was heated up to 240°C (3 zones heated to 195°C, 220°C and 240°C respectively), the sheath extruder was heated up to 235°C (3 zones heated to 200°C, 215°C and 235°C respectively). The spinning beam temperature was set to 240°C. Polymer throughput was set to 0,25 g/min/capillary. Filaments were cooled with an air temperature of 20°C. The inlet pressure is shown in Table 2. Fibers were collected on a running belt; the batt gsm was set to 130 g/m². The batt from the belt was cut into test samples of the size 10 x 7 cm. The samples were carefully moved to a separate oven and activated for a period of 5 minutes at a set temperature. These temperatures are also shown in Table 2.

*Table 2:*

| Example | 1A | 1B | 1C | 1D | 1E | 1F |
|---|---|---|---|---|---|---|
| material composition | PLA/PP | | | | | |
| oven temperature [0 C] | 100°c | | 120°c | | 140°C | 160°C |
| inlet pressure [kPa[ | 100 | 50 | 100 | 150 | 100 | 100 |
| draw down ratio | 215 | 215 | 215 | 202 | 215 | 215 |
| filament speed [m/min] | 4 889 | 4 884 | 4 889 | 4 582 | 4 889 | 4 889 |
| activation change in fabric thickness | +100% | +60% | +133% | +96% | +155% | +137% |
| activation change in fabric length | -3% | -5% | -5% | -4% | -3% | -6% |
| activation change in fabric width | -3% | -3% | -2% | -2% | -4% | -3% |
| resilience * 100% | 37 | 27 | 35 | 23 | 36 | 34 |

(continued)

| Example | 1A | 1B | 1C | 1D | 1E | 1F |
|---|---|---|---|---|---|---|
| recovery * 100% | 98 | 98 | 98 | 97 | 98 | 98 |
| compressed absolute void volume[m$^3$/kg] | 0,02384 | 0,02684 | 0,02711 | 0,02511 | 0,02505 | 0,02654 |
| Structural softness [m$^4$mm$^2$g$^{-2}$] | 568 | 378 | 641 | 254 | 587 | 578 |
| Void volume softness index VVSI | 13,53 | 10,14 | 17,37 | 6,38 | 14,71 | 15,35 |

Examples 1A, 1C and 1 D demonstrate the possibility of controlling the shrinkage level by the size of fiber drawing force (inlet pressure). The cooling was the same for all three examples. Not to be bound by theory, we believe that the drawing force can help to induce a range of semistable crystalline states of the filament, some of which are more desirable for increasing thickness than others. When the drawing force is weaker, the resulting fiber can provide a relatively low toughness, which can then result in a lower final thickness of the web. On the other hand, when the drawing force is strong, the induced crystallization is set so that its volume changes and thus the shrinkage force during activation is lower, which again results in a lower final thickness. When the drawing force is just right, as shown in the example 1 C, the final fabric thickness and also the structural softness is the highest. A person skilled in the art will realize the possibility of finding the optimal condition by tuning the filament speed and the draw down ratio.

Examples 1 A, 1 C, 1 E and 1 F present the possibility to control the shrinkage level by the activation temperature. For these exact conditions, it can be seen that the best final thickness has the sample activated at 140°C (+155%), however, the material is complex and the key evaluation parameter are structural softness and VVSI, the very best sample being activated at 120°C.

**Example 2,** - thereof 2A comparative, not part of the invention

The nonwoven acquisition fabric consists of bicomponent filaments with a noncrimpable cross-section layout, core/sheath type, where the core/sheath mass ratio is 70:30, the core is formed using PET (Type 5520 resin from Invista) and the sheath is formed using PP (Tatren HT 2511 from Slovnaft). The nonwoven was produced on a laboratory line at UTB Zlfn, Centre of Polymer Systems. The core extruder was heated up to 340°C (3 zones heated to 340°C, 335°C and 325°C respectively), the sheath extruder was heated up to 235°C (3 zones heated to 200°C, 215°C and 235°C respectively). The spinning beam temperature was set to 305°C. Polymer throughput was set to 0,25 g/min/capillary. Filaments were cooled with an air temperature of 20°C. The inlet pressure is shown in Table 3. Fibers were collected on a running belt; the batt gsm was set to 75 g/m$^2$. The batt from the belt was cut into test samples of the size 10 x 7 cm. Samples were carefully moved to a separate oven and activated for 5 minutes at a set temperature. The temperatures are shown in Table 3.

**Example 3**

The nonwoven acquisition fabric consists of bicomponent filaments with a noncrimpable cross-section layout, core/sheath type, where the core/sheath mass ratio is 70:30, the core is formed using PET (Type 5520 resin from Invista) and the sheath is formed using a blend of 95% PP (Tatren HT 2511 from Slovnaft) and 5% of white masterbatch (CC10084467BG from PolyOne). The nonwoven was produced on a laboratory line at UTB Zlfn, Centre of Polymer Systems. The core extruder was heated up to 340°C (3 zones heated to 340°C, 335°C and 325°C respectively), the sheath extruder was heated up to 235°C (3 zones heated to 200°C, 215°C and 235°C respectively). The spinning beam temperature was set to 305°C. Polymer throughput was set to 0,25 g/min/capillary. Filaments were cooled with an air temperature of 20°C. The inlet pressure is shown in Table 3. Fibers were collected on a running belt; the batt gsm was set to 75 g/m$^2$. The batt from the belt was cut into test samples of the size 10 x 7 cm. Samples were carefully moved to a separate oven and activated for 5 minutes at a set temperature. The temperatures are shown in Table 3.

**Example4**

The nonwoven acquisition fabric consists of bicomponent filaments with a noncrimpable cross-section layout, core/sheath type, where both core and sheath were formed using PET (Type 5520 resin from Invista). The nonwoven was produced on a laboratory line at UTB Zlfn, Centre of Polymer Systems. The extruders were heated up to 340 °C (3 zones heated to 340°C, 335°C and 325°C respectively). The spinning beam temperature was set to 305°C. Polymer throughput was set to 0,25 g/min/capillary. Filaments were cooled with an air temperature of 20°C. The inlet pressure is shown in Table 3. Fibers were collected on a running belt; the batt gsm was set to 75 g/m$^2$. The batt from the belt was cut into test samples ofthe size 10x 7 cm. Samples were carefully moved to a separate oven and activated for 5 minutes at a set temperature. The temperatures are shown in Table 3.

Table 3:

| Example | 2A | 2B | 2C | 2D | 2E | 2F | 3 | 4 |
|---|---|---|---|---|---|---|---|---|
| material composition | PET/PP | | | | | | PET/ (PP+ white) | PET/ PET |
| oven temperature [°C] | 100°C | 120°C | 140°C | 150°C | 160°C | 140°C | | |
| inlet pressure [kPa] | 50 | | | | | 100 | 100 | 100 |
| draw down ratio | 214 | 214 | 214 | 214 | 214 | 224 | 224 | 253 |
| filament speed [m/min] | 4 469 | 4 469 | 4 469 | 4 469 | 4 469 | 4 696 | 4 088 | 4 766 |
| activation change in fabric thickness | +19% | +31% | +51% | +47% | +39% | +103 % | +105% | +222% |
| activation change in fabric length | -5% | -6% | -8% | -8% | -7% | -15% | -14% | -63% |
| activation change in fabric width | -5% | -2% | -5% | -6% | -6% | -15% | -15% | -63% |
| resilience * 100% | 2 | 33 | 33 | 23 | 12 | 40 | 41 | - |
| recovery * 100% | 95 | 97 | 98 | 98 | 98 | 98 | 98 | - |
| compressed absolute void volume [m$^3$/kg] | 0,0246 2 | 0,01992 | 0,02030 | 0,02341 | 0,02334 | 0,01830 | 0,01781 | - |
| Structural softness [m$^4$mm$^2$g$^{-2}$] | 13 | 306 | 321 | 223 | 88 | 397 | 400 | - |
| Void volume softness index VVSI | 0,31 | 6,10 | 6,51 | 5,22 | 2,07 | 7,27 | 7,12 | |

Examples 2C and 3 demonstrate the same principle as examples 1B-1D above. Examples 2A-2F demonstrate the possibility of controlling the shrinkage level using the activation temperature. For these exact conditions, it is evident that the best final thickness was on the sample activated at 140°C (+51%) and the same temperature was also the best from the structural softness point of view. Example 3 and comparative example 4 demonstrate the irnportance of correct sheath material in the material. It can be clearly seen, that the PET/PET material has a significantly different behavior during activation, which leads to a different shrinkage level (see Fig. 11). Example 3 increased its volume by 47% and also provided good resilience and recovery values. In contrast, the PET/PET sample had decreased in volume by -56% and shrunk into a hard slightly bent piece, on which it was not possible to measure the resilience or recovery values. It shall be noted, that the CD and MD shrinkage level is below 10 percent for examples 2A-F, as is the case with the abovementioned examples A-E. In contrast, the increase of thickness is much higher than the decrease in the CD and MD directions. Examples 2F and 3 provide very good values of structural softness and also an acceptable level of CD and MD shrinkage (15%). It shall be also noted, that the PET used in examples 2-4 contained a small amount of Ti02 (used as a mating agent by the polymer producer). In contrast, the PLA used in example 1 does not contain any Ti02. One layer or two layers can be produced inline, for example, on a Reifenhäuser Reicofil pilot line at Troisdorf, Germany. This line was used to produce the nonwovens described in examples 5, 6, 8, 9, 10, 11 with following standard setting: Pre-consolidation air speed 2,3 [m/s]; Activation air speed 1,3 [m/s]; Bonding air speed 1,3 [m/s]; Quenching air temperature 20 [°C]. The pilot line is equipped with two BiCo spunbond beams, each of them equipped with two extruders supplying a BiCo coat hanger die. The extrusion system allows temperatures up to 350°C to process a wide variety of polymers within a specific total throughput range per beam of 80 to 450kg/h/m. Multiple spinnerets are available with different capillary densities as well as capillary geometries. Spin packs having a HILLS melt distribution system are used to form in addition to the standard cross section described in this invention almost every cross section to imagine on a 1,1 m wide spinneret. The devices for cooling, stretching and formation are today's industry-reference covering a wide range of cooling and drawing conditions ensuring an excellent uniform filament batt. The forming belt runs up to 400m/min production speed. The nonwoven layer from the first spunbond beam passes optional pre-consolidation, activation and/or bonding devices inline before the layer from the second beam is stacked on top of the first one. The second beam is equipped with similar inline-equipment for optional pre-consolidation, activation and bonding as the first one. The pre- or finally bonded product is wound up on an inline slitter-winder or might be inline bonded on a drum bonder prior to winding. Surfactant treatment by a kiss roll to modify surface properties of the nonwoven is available inline or offline.

Example5

The nonwoven was produced on one bicomponent spunbond beam round shape core/sheath type. The core/sheath mass ratio was 70/30. The core was produced from PET (Type 5520 resin from Invista) and the sheath was produced using PE (ASPUN 6834 from Dow). The process conditions and final fabric parameters are shown in the Table 4 below. The activation and the bonding were done on a single piece of equipment with a set activation and bonding zone.

*Table 4:*

| Example | 05A | 05B | 05C | 05D |
|---|---|---|---|---|
| Composition polymer plastic group A / polymer plastic group B | PET /PE | PET /PE | PET /PE | PET /PE |
| BiCo cross section polymer plastic group A / polymer plastic group B | C/S | C/S | C/S | C/S |
| layer count | 1 | 1 | 1 | 1 |
| capillary shape | round | round | round | round |
| spinneret capillary density [1000/m] | 1,1 | 1,1 | 1,1 | 1,1 |
| melt temperature polymer plastic group A [°C] | 286 | 286 | 286 | 286 |
| melt temperature polymer plastic group B [°C] | 267 | 267 | 267 | 267 |
| drawing force level | very low | low | medium | high |
| suction force level | low | medium | medium | medium |
| cooling air / polymer ratio | 35,2 | 37,9 | 41 | 42,7 |
| draw down ratio | 443 | 483 | 625 | 665 |
| filament speed [m/min] | 3496 | 3810 | 4930 | 5253 |
| pre-consolidation time [s/1000] | 68 | 68 | 68 | 68 |
| pre-consolidation temperature [°C] | 130 | 130 | 130 | 130 |
| activation time [s/1000] | 682 | 682 | 682 | 682 |
| activation temperature [°C] | 135 | 135 | 135 | 135 |
| bonding time [s/1000] | 2455 | 2455 | 2455 | 2455 |
| bonding temperature [°C] | 130 | 130 | 130 | 130 |
| Basis weight [gsm] | 62 | 59 | 64 | 64 |
| Apparent fiber diameter [$\mu$m] | 38 | 36 | 32 | 31 |
| Resilience * 100% | 35 | 37 | 37 | 35 |
| Compressibility [mm]/basis weight [gsm] | 0,0111 | 0,0113 | 0,0101 | 0,0089 |
| Recovery * 100% | 99 | 98 | 99 | 99 |
| Thickness [mm] | 1,96 | 1,83 | 1,72 | 1,63 |
| compressed absolute void volume [$m^3$/kg] | 0,01971 | 0,01892 | 0,01598 | 0,01576 |
| Structural softness [$m^4mm^2g^{-2}$] | 346 | 342 | 270 | 224 |
| Void volume softness index VVSI | 6,84 | 6,49 | 4,29 | 3,54 |

Examples 5A - D demonstrate the importance of the cooling air / polymer ratio, draw down ratio and filament speed on the final fabric properties. It can be seen that with increased drawing and cooling, the fabric thickness, the fiber diameter and also the Structural softness and the VVSI decrease. On the other hand, the mechanical properties of the final product increase. A person skilled in the art will realize, based on the final application, which settings are the best for the product.

Example 6

The nonwoven acquisition fabric was produced on two subsequent bicomponent spunbond beams, round shape core-sheath type. The core/sheath mass ratio was 70/30. The core was produced from PET (Type 5520 resin from Invista) and the sheath was produced using PE (ASPUN 6834 from Dow). The process conditions and final fabric parameters are shown in Table 5 below. Activation and bonding were done on a single piece of equipment with a set activation and bonding zone.

The assumed in-use performance of nonwoven acquisition fabrics 6A-6C has been tested by an absorbent incontinence slip that utilized nonwoven acquisition fabrics 6A-6C in the examples described herein. The design of the absorbent incontince slip is described in more detail as follows.

The absorbent incontinence slip comprised a topsheet SMS nonwoven layer, PP, hydrophilic durable, basis weight 12 g/m$^2$, purchased from Avgol Nonwovens. The respective nonwoven acquisition fabric 6A, 6B or 6C was used as acquisition component, disposed directly below the topsheet. It had a rectangular shape: length of 270 mm, width of 100 mm. An absorbent storage core was disposed directly below the acquisition component. The absorbent storage core consisted of an upper layer and a lower layer. The lower layer was of hourglass shape and had a length of 750 mm and a width of 270 mm (in the front and back) and a width of 130 mm in the crotch , respectively, and it comprised of 34,8 g cellulose fluff pulp fibers only, purchased from Resolute Forest Products as CoosAbsorb®S. Basis weight: 250 g/m$^2$. The upper layer had a rectangular shape and had a length of 460 mm and a width of 130 mm and comprised of a mixture of 38,8 g of the same cellulose fluff pulp fibers as the lower layer and 14,5 g superabsorbent polymer, purchased from Evonik Nutrition & Care GmbH as SXM 9791.

The upper layer had a single channel, i.e. an area free of absorbent material. Said channel had a length of 130 mm and a width of 10 mm and positioned in a central area along the longitudinal axis of the upper layer, at a distance from the front edge of the upper layer of 90 mm.

A water impermeable backsheet (film-nonwoven laminate) was disposed directly below the lower layer of the absorbent storage core.

The rewet and acquisition times according to the test method described below was recorded and listed in table 5.

*Table 5:*

| Example | 06A | 06B | 06C | 06D |
|---|---|---|---|---|
| Composition polymer plastic group A / polymer plastic group B | PET / PE | PET / PE | PET / PE | PET / PE |
| BiCo cross section polymer plastic group A / polymer plastic group B | C/S | C/S | C/S | C/S |
| layer count | 1 | 1 | 1 | 1 |
| capillary shape | round | round | round | round |
| spinneret capillary density [1000/m] | 1,1 | 1,1 | 1,1 | 1,1 |
| melt temperature polymer plastic group A [°C] | 281 | 281 | 281 | 281 |
| melt temperature polymer plastic group B [°C] | 266 | 266 | 266 | 266 |
| drawing force level | medium | medium | medium | medium |
| suction force level | low | low | low | low |
| cooling air / polymer ratio | 38 | 38 | 37,9 | 37,9 |
| draw down ratio | 465 | 478 | 551 | 517 |
| filament speed [m/min] | 3669 | 3773 | 4343 | 4048 |
| pre-consolidation temperature [°C] | 130 | 130 | 130 | 130 |
| pre-consolidation time [s/1000] | 35 | 23 | 17 | 15 |

| Example | 06A | 06B | 06C | 06D |
|---|---|---|---|---|
| activation time [s/1000] | 349 | 231 | 169 | 150 |
| activation temperature [°C] | 135 | 135 | 135 | 135 |
| bonding time [s/1000] | 1256 | 831 | 610 | 540 |
| bonding temperature [°C] | 130 | 130 | 134 | 134 |
| Basis weight [gsm] | 60 | 40 | 30 | 26 |
| Apparent fiber diameter [$\mu$m] | 37 | 36 | 34 | 35 |
| Resilience * 100% | 32 | 35 | 19 | 11 |
| Compressibility [mm]/basis weight [gsm] | 0,0104 | 0,0122 | 0,0067 | 0,0035 |
| Recovery * 100% | 99 | 97 | 97 | 97 |
| Thickness [mm] | 1,9 | 1,4 | 1 | 0,9 |
| MD/CD tensile ratio | 1,23 | 0,89 | 1,14 | 1,21 |
| compressed absolute void volume [$m^3$/kg] | 0,02107 | 0,02162 | 0,02699 | 0,0286 |
| Structural softness [$m^4mm^2g^{-2}$] | 331 | 407 | 225 | 110 |
| Void volume softness index VVSI | 6,97 | 8,81 | 6,09 | 3,14 |
| Rewet [g] | 0,9 | 1,7 | 1,9 | |
| Acquisition time 1st gush [s] | 41 | 45 | 49 | |
| Acquisition time 2nd gush [s] | 31 | 32 | 37 | |
| FRW [$g^2/m^2$) | 55 | 68 | 58 | |

The nonwoven acquisition fabric 06E (see table 5b) was produced as decribed in Example 6, however as a dual layer fabric. If different, the process parameters are given for each of the layers. The assumed in-use performance (Rewet) was tested as described in Example 6.

Table 5b:

| Example | 06E |
|---|---|
| Composition polymer plastic group A / polymer plastic group B | PET / PE |
| BiCo cross section polymer plastic group A / polymer plastic group B | C/S |
| layer count | 1 |
| capillary shape | round |
| spinneret capillary density<br>upper layer A<br>lower layer B [1000/m] | 1,1<br>3,2 |
| melt temperature polymer plastic group A , upper layer A [°C]<br>"-" lower layer B [°C] | 281<br>282 |
| melt temperature polymer plastic group B , upper layer A [°C]<br>"-" lower layer B [°C] | 266<br>265 |
| drawing force level upper layer A<br>"-" lower layer B | medium<br>low |
| suction force level upper layer A<br>"-" lower layer B | low<br>low |

| Example | 06E |
|---|---|
| draw down ratio upper layer A <br> -"- lower layer B | 391 <br> 360 |
| filament speed upper layer A <br> -"- lower layer B [m/min] | 3108 <br> 3884 |
| pre-consolidation time [s/1000] | 48,4 |
| pre-consolidation temperature [°C] | 130 |
| activation time [s/1000] | 7245 |
| activation temperature [°C] | 130 |
| bonding time [s/1000] | 30 |
| bonding temperature [°C] | 130 |
| Basis weight [gsm] | 83 |
| Apparent fiber diameter [$\mu$m] <br> Upper layer A <br> Lower layer B | <br> 40 <br> 24 |
| Resilience * 100% | 28 |
| Compressibility [mm]/basis weight [gsm] | 0,0067 |
| Recovery * 100% | 99 |
| Thickness [mm] | 2 |
| MD/CD tensile ratio | 0,93 |
| compressed absolute void volume [$m^3$/kg] | 0,0162 |
| Structural softness [$m^4 mm^2 g^{-2}$] | 159 |
| Void volume softness index VVSI | 2,58 |
| Rewet [g] | 0,7 |
| Acquisition time 1st gush [s] | 37 |
| Acquisition time 2nd gush [s] | 29 |
| FRW [$g^2/m^2$) | 58 |

Example 7 - comparative, not part of the invention

The nonwoven acquisition fabric consists of bicomponent filaments with a crimpable cross-section layout, round eccentric core/sheath type, where the core is from PET and sheath from PE. The fabric was hot-air bonded. The fabric parameters are shown in Table 6 below.

*Table 6:*

| Example | 07A | 07B | 07C |
|---|---|---|---|
| Composition <br> polymer plastic group A / polymer plastic group B | PET/PE | PET / PE | PET / PE |
| BiCo cross section <br> polymer plastic group A / polymer plastic group B | eC/S | eC/S | eC/S |
| layer count | 1 | 1 | 1 |
| capillary shape | round | round | round |
| Basis weight [gsm] | 51 | 83 | 33 |
| Resilience * 100% | 15 | 17 | 17 |

(continued)

| Example | 07A | 07B | 07C |
|---|---|---|---|
| Compressibility [mm]/basis weight [gsm] | 0,0028 | 0,0033 | 0,0036 |
| Recovery * 100% | 98 | 99 | 97 |
| Thickness [mm] | 0,9 | 1,6 | 0,7 |
| MD/CD tensile ratio | 5,31 | 2,22 | 3,19 |
| compressed absolute void volume [m$^3$/kg] | 0,0168 | 0,0153 | 0,0153 |
| Structural softness [m$^4$mm$^2$g$^{-2}$] | 52 | 63 | 74 |
| Void volume softness index VVSI | 0,87 | 0,96 | 1,13 |

Examples 7A - C represent a one-layer nonwoven acquisition fabrics with comparable polymer composition. However, the fibers have a crimpable crossection and crimps, as is visible on the fabric cross-section in Fig. 15 and also it can be compared with the cross-sections of examples according to 5A+5D - Fig. 16. The structural softness, void volume and VVSI of the examples are, compared to examples 5 and 6 significantly lower, as well as the fabric thickness.

Example 8

The nonwoven acquisition fabric was produced from two subsequent bicomponent spunbond beams, round shape core-sheath type. The core/sheath mass ratio was 70/30. The core was produced from PET (Type 5520 resin from Invista) and the sheath was produced using coPET (type 701k from Invista). The process conditions and final fabric parameters are shown in Table 7 below. The activation and the bonding were performed inline on the belt with separate devices for activation and bonding.

Example 9, - thereof 9A comparative, not part of the invention

The nonwoven acquisition fabric was produced on two subsequent bicomponent spunbond beams, round shape core-sheath type. The core/sheath mass ratio was 70/30. The core was produced from PET (Type 5520 resin from Invista) and the sheath was produced using coPET (type 701k from Invista). The process conditions and final fabric parameters are shown in Table 7 below. The activation was performed in a single step, the bonding in a second step on different equipment. In the case of examples 9A + B, the bonding was done immediately after activation inline on a drum. In the case of Example 9C, the bonding was done on different equipment several days after activation offline on a drum.

*Table 7:*

| Example | 08A | 08B | 09A | 09B | 09C |
|---|---|---|---|---|---|
| Composition polymer plastic group A / polymer plastic group B | PET / CoPET | PET / Co-PET | PET / CoPET | PET / CoPET | PET / Co-PET |
| BiCo cross section polymer plastic group A / polymer plastic group B | C/S | C/S | C/S | C/S | C/S |
| layer count | 1 | 1 | 1 | 1 | 1 |
| capillary shape | round | round | round | round | round |
| spinneret capillary density [1000/m] | 1,1 | 1,1 | 1,1 | 1,1 | 1,1 |
| melt temperature polymer plastic group A [°C] | 279 | 279 | 276 | 275 | 279 |
| melt temperature polymer plastic group B [°C] | 276 | 276 | 275 | 275 | 276 |
| drawing force level | high | high | high | high | high |
| suction force level | high | high | medium | medium | high |
| cooling air / polymer ratio | 41,1 | 41,1 | 41,1 | 41,1 | 41,1 |
| draw down ratio | 675 | 630 | 649 | 619 | 711 |
| filament speed [m/min] | 4846 | 4521 | 4656 | 4441 | 5104 |

(continued)

| Example | 08A | 08B | 09A | 09B | 09C |
|---|---|---|---|---|---|
| pre-consolidation time [s/1000] | 38 | 50 | 46 | 35 | 25 |
| pre-consolidation temperature [°C] | 160 | 160 | 160 | 160 | 160 |
| activation time [s/1000] | 375 | 500 | 462 | 349 | 250 |
| activation temperature [°C] | 140 | 140 | 150 | 150 | 140 |
| bonding time [s/1000] | 1350 | 1800 | 1522 | 1151 | 5376 |
| bonding temperature [°C] | 155 | 155 | 215 | 219 | 218 |
| Basis weight [gsm] | 59 | 79 | 76 | 58 | 38 |
| Apparent fiber diameter [$\mu$m] | 31 | 32 | 31 | 32 | 30 |
| Resilience * 100% | 28 | 23 | 12 | 16 | 17 |
| Compressibility [mm]/basis weight [gsm] | 0,0086 | 0,0063 | 0,0023 | 0,0037 | 0,0046 |
| Recovery * 100% | 100 | 99 | 100 | 100 | 97 |
| Thickness [mm] | 1,8 | 2,2 | 1,5 | 1,3 | 1 |
| MD/CD tensile ratio | 0,93 | 0,84 | 1,62 | 1,41 | 1,2 |
| compressed absolute void volume [$m^3$/kg] | 0,02162 | 0,0202 | 0,01683 | 0,01825 | 0,02118 |
| Structural softness [$m^4mm^2g^{-2}$] | 269 | 168 | 46 | 82 | 118 |
| Void volume softness index VVSI | 5,81 | 3,53 | 0,77 | 1,49 | 24,9 |
| Rewet [g] | | | | 1,1 | |
| Acquisition time 1st gush [s] | | | | 33 | |
| Acquisition time 2nd gush [s] | | | | 25 | |
| FRW [$g^2$/$m^2$) | | | | 64 | |

Examples 8 and 9 demonstrate the importance of the bonding conditions. Example 8 focuses on high structural softness. The sheath polymer melting temperature is higher than in previous examples, thus the optimal bonding temperature is higher than the core activation temperature. The used bonding temperature (155°C) is close to the optimal activation temperature, not reaching the optimal bonding level. Also the mechanical properties are at a lower level. In contrast, example 9 focuses on the optimal bonding level with better mechanical properties, but lower thickness and structural softness. Example 9C was bonded offline, simulating the capability to separate the process steps "activation" and "bonding" from each other. After activation the product is wound up and transported to another location. There it is unwound and the final bonding is done. A person skilled in the art will realize that apart from bonding temperature and time, especially the pressure during the bonding phase (including the pressure from the air throughput, the pressure from the tension of the nonwoven web, the pressure from auxiliary guide rollers, etc.) can also influence the final thickness and reduce the structural softness. The difference between examples 9A+B and 9C represents not only the inline/offline possibility, but also the influence of different bonding settings. The assumed in-use performance of nonwoven acquisition fabric 9B has been tested by testing an absorbent incontinence slip that utilized nonwoven acquisition fabric 9B as acquisition component. The design of the absorbent incontinence slip was the same as described above in the context of Example 6.

Example 10

The nonwoven acquisition fabric was produced on two subsequent bicomponent spunbond beams, trilobal shape core-sheath type. The core/sheath mass ratio was 70/30. The core was produced from PET (Type 5520 resin from Invista) and the sheath was produced using coPET (type 701k from Invista). The process conditions and final fabric parameters are shown in Table 8 below. The activation and the bonding were performed on a single piece of equipment with a set activation and bonding zone.

Example 11

The nonwoven acquisition fabric was produced on two subsequent bicomponent spunbond beams, round shape core-sheath type. The core/sheath mass ratio was 70/30. The core was produced from PET (Type 5520 resin from Invista) and the sheath was produced using coPET (Type RT5032 from Trevira). The process conditions and final fabric parameters are shown in Table 8 below. The activation and the bonding were performed on a single piece of equipment with a set activation and bonding zone.

*Table 8:*

| Example | 10 | 11A | 11B |
|---|---|---|---|
| Composition polymer plastic group A / polymer | PET / CoPET | PET / CoPET | PET / CoPET |
| BiCo cross section polymer plastic group A /polymer plastic group B | C/S | C/S | C/S |
| layer count | 1 | 1 | 1 |
| capillary shape | trilobal | round | round |
| spinneret capillary density [1000/m] | 1,1 | 3,21 | 3,21 |
| melt temperature polymer plastic group A [°C] | 276 | 299 | 300 |
| melt temperature polymer plastic group B [°C] | 275 | 277 | 278 |
| drawing force level | high | low | low |
| suction force level | medium | low | low |
| cooling air / polymer ratio | 38,7 | 28,1 | 28,2 |
| draw down ratio | 1947 | 386 | 403 |
| filament speed [m/min] | 3607 | 3759 | 3925 |
| pre-consolidation time [s/1000] | 92 | 56 | 42 |
| pre-consolidation temperature [°C] | 120 | 130 | 130 |
| activation time [s/1000] | 923 | 561 | 417 |
| activation temperature [°C] | 145 | 130 | 130 |
| bonding time [s/1000] | 3323 | 2019 | 1500 |
| bonding temperature [°C] | 140 | 130 | 130 |
| Basis weight [gsm] | 56 | 60 | 80 |
| Apparent fiber diameter [$\mu$m] | 42 | 23 | 22 |
| Resilience * 100% | 39 | 25 | 23 |
| Compressibility [mm]/basis weight [gsm] | 0,0125 | 0,0081 | 0,007 |
| Recovery * 100% | 98 | 98 | 99 |
| Thickness [mm] | 1,8 | 1,9 | 2,4 |
| MD/CD tensile ratio | 1,13 | 0,68 | 0,69 |
| compressed absolute void volume [$m^3$/kg] | 0,01888 | 0,02306 | 0,02204 |
| Structural softness [$m^4mm^2g^{-2}$] | 395 | 253 | 204 |
| Void volume softness index VVSI | 7,46 | 5,84 | 4,5 |
| Rewet [g] | | | 0,6 |
| Acquisition time 1st gush [s] | | | 32 |
| Acquisition time 2nd gush [s] | | | 27 |
| FRW [$g^2$/$m^2$) | | | 48 |

Example 10 demonstrates the possibility of using filaments with a different non-round shape. Example 11A and 11B demonstrates the possibility of also using a spinneret with higher capillary density. The assumed in-use performance of nonwoven acquisition fabric 11B has been tested by testing an absorbent incontinence slip that utilized nonwoven acquisition fabric 11B as acquisition component. The design of the absorbent incontinence slip was the same as described above in the context of Example 6.

Example 11C

The nonwoven acquisition fabric was produced as decribed in Example 11, however as a dual layer nonwoven fabric. If different, the process parameters are given for each of the layers.. The assumed in-use performance was tested as described in Example 6.

Table 8b:

| Example | 11C |
|---|---|
| Composition<br>polymer plastic group A / polymer plastic group B | PET / coPET |
| BiCo cross section polymer plastic group A / polymer plastic group B | C/S |
| layer count | 2 |
| capillary shape | round |
| spinneret capillary density upper layer A<br>lower layer B [1000/m] | **1,1**<br>3,2 |
| melt temperature polymer plastic group A , upper layer A [°C]<br>"-" lower layer B [°C] | 281<br>282 |
| melt temperature polymer plastic group B , upper layer A [°C]<br>"-" lower layer B [°C] | 266<br>265 |
| drawing force level upper layer A<br>"-" lower layer B | medium<br>low |
| suction force level upper layer A<br>"-" lower lay B | medium<br>low |
| draw down ratio upper layer A -"- lower layer B | 684<br>382 |
| filament speed upper layer A -"- lower layer B [m/min] | 4892<br>3708 |
| pre-consolidation time [s/1000] | 58,2 |
| pre-consolidation temperature [°C] | 130 |
| activation time [s/1000] | 582 |
| activation temperature [°C] | 130 |
| bondingtime [s/1000] | 2095 |
| bonding temperature [°C] | 130 |
| Basis weight [gsm] | 58 |
| Apparent fiber diameter [μm]<br>Upper layer A<br>Lower layer B | <br>31<br>23 |
| Resilience * 100% | 26 |
| Compressibility [mm]/basis weight [gsm] | 0,0078 |
| Recovery * 100% | 100 |
| Thickness [mm] | 1,7 |

(continued)

| Example | 11C |
|---|---|
| MD/CD tensile ratio | 0,74 |
| compressed absolute void volume[$m^3$/kg] | 0,02165 |
| Structural softness [$m^4mm^2g^{-2}$] | 232 |
| Void volume softness index VVSI | 5,02 |
| Rewet [g] | 0,8 |
| Acquisition time 1st gush [s] | 39 |
| Acquisition time 2nd gush [s] | 31 |
| FRW [$g^2$/$m^2$) | 46 |

Example 12 - carded nonwovens, - comparative, not part of the invention A first set of nonwoven samples chosen as examples representing carded nonwovens were produced by TWE group under the brand "TWE Hygiene". The nonwoven consists of short bicomponent fibers, core sheath type, where the core is from PET and the sheath from PE. The fabric was produced using carding technology, consolidated by hot-air-bonding. The fabric parameters are shown in Table 9 below.

Table 9:

| Example | 12A | 128 | 12C |
|---|---|---|---|
| Composition polymer plastic group A/ polymer plastic group B | Probably PET/PE | Probably PET/PE | Probably PET/PE |
| Fabric name | TL 7 | TL 1 | TWE 286 |
| Technology | carded | carded | carded |
| Basis weight [gsm] | 97 | 67 | 23 |
| Resilience * 100% | 41 | 44 | 35 |
| Compressibility [mm]/basis weight [gsm] | 0,0082 | 0,0082 | 0,0065 |
| Recovery * 100% | 99 | 99 | 98 |
| Thickness [mm] | 1,9 | 1,2 | 0,4 |
| MD/CD tensile ratio | 5,18 | 7,83 | 3,15 |
| compressed absolute void volume[$m^3$/kg] | 0,0109 | 0,0096 | 0,0113 |
| Structural softness [$m^4mm^2g^{-2}$] | 161 | 151 | 120 |
| Void volume softness index VVSI | 1,75 | 1,46 | 1,36 |

Example 13 - carded nonwoven, - comparative, not part of the invention Another nonwoven sample chosen as an example representing carded nonwovens is listed in Table 10. The nonwoven consisted of two types of fibers (PET and Co PET). The assumed in-use performance of the carded nonwoven 13 has been tested by testing an absorbent incontinence slip that utilized nonwoven fabric 13 as acquisition component. The design of the absorbent incontinence slip was the same as described above in the context of Example 6.

Table 10:

| Example | 13 |
|---|---|
| Polymer composition Fibers of type 1 Polymer composition Fibers of type 2 | PET CoPET |
| Technology | carded |
| Basis weight [g/$m^2$] | 40 |

(continued)

| Example | 13 |
|---|---|
| Resilience * 100% | 19 |
| Compressibility [mm]/basis weight [g/m$^2$sm] | 0,0020 |
| Recovery * 100% | 99 |
| Thickness [mm] | 0,4 |
| MD/CD tensile ratio | 6,86 |
| compressed absolute void volume[m$^3$/kg] | 0,0078 |
| Structural softness [m$^4$mm$^2$g$^{-2}$] | 21 |
| Void volume softness index VVSI | 0,17 |
| Rewet [g] | 5,2 |
| Acquisition time 1$^{st}$ gush [s] | 48 |
| Acquisition time 2nd gush [s] | 31 |

Example 14 - Crosslinked Cellulose material, - comparative, not part of the invention Another sample chosen as an example representing an already established solution is listed in Table 11.

The assumed in-use performance of the intra crosslinked cellulose material has been tested by testing an absorbent incontinence slip that utilized the crosslinked cellulose material purchased from International Paper as CMC 520 as acquisition component. The design of the absorbent incontinence slip is almost the same as described above in the context of Example 6. Different from that: 21,5 g comminuted cellulose fluff pulp fibers in the upper layer of the absorbent storage core (instead of 23,5 g) resulting in a basis weight of 358 g/m$^2$ of said upper layer.

*Table 11:*

| Example | 14 |
|---|---|
| Intra-crosslinked cellulose fibers CMC 520 | |
| Technology | airfelt |
| Basis weight [g/m$^2$] | 250 |
| Rewet [g] | 11,1 |
| Acquisition time 1$^{st}$ gush [s] | 48 |
| Acquisition time 2nd gush [s] | 33 |
| FRW [g$^2$/m$^2$) | 2775 |

A person skilled in the art understands that modern carded materials or crosslinked cellulose fiber material (CF), as a result of long-term development, may also be suitable. On the other hand, carded materials are produced from staple fibers and the high number of ends of these fibers across and along a nonwoven layer may be unwelcome for certain applications. Example 12A -12C and 13 provides the properties of four commercially available carded fabrics specified for use in hygiene. Comparing this set of carded nonwovens with the referenced nonwoven acquisition fabric samples with comparative polymer compositions, it can be seen that the carded materials typically have a slightly higher resilience (12A-12C), but due to their lower thickness, their compressibility is comparable or lower, so the real tough and feel of soft-loft is equal or better for the referenced nonwoven acquisition fabrics. Likewise the CF material shows less performance as to the liquid management of the absorbent article let alone that the CF material requires airfelt technology in addition to the technology for producing the fibers as such.

Testing methodology and further definitions

Rewet and Acquisition time 1st gush [s] and 2nd gush [s] are determined as follows:

Principle

This testing method determines:

a) The moisture return of the absorbent article by measuring its capacity to retain an amount of synthetic urine without it being possible to extract it externally, under specific conditions of time and pressure.
b) The rate of absorption of each gush

**Instruments and apparatus**

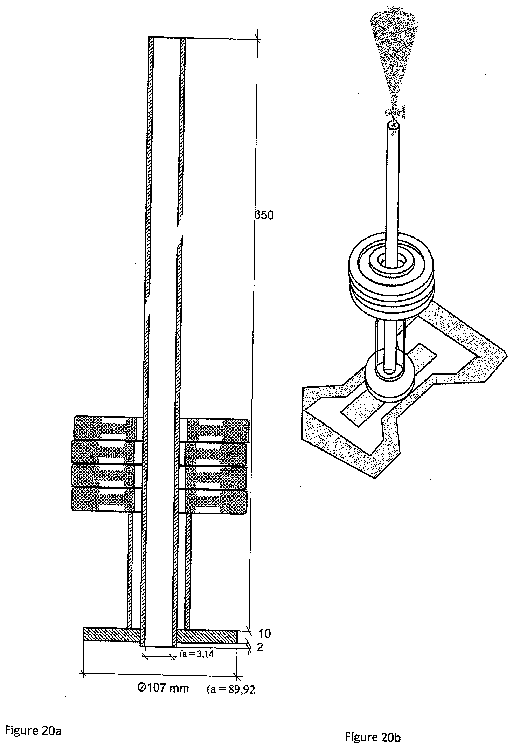

- A 250 ml test tube with graduation to 150 ml
- 250 ml pear-shaped separatory funnel funnel with pouring speed of 16 ml/s.
- Timers
- Transparent methacrylate column made up of a tube with interior diameter of 21 mm by 650 mm of length and with a circular base 10 mm thick with a diameter of 107 mm (see figure 20a).
- Cast iron weights of ½ kg each. Total weight (adding the weight of the column to the weight of the separator tube) is 3,7 kg (disks with central opening). The total pressure to be put on the absorbent article is 0,585 psi.
- Separator tube for the weights in transparent methacrylate, 40 mm in interior diameter by 30 mm high in order to be able to view the descent of the liquid down the tube, only if necessary.
- Laboratory stand with fastening clips and arms.
- Paper filter disks 90 mm in diameter, with the following characteristics: Grammage: 90 $g/m^2$; thickness: 0,199 mm, filtration 261 s/100 ml as referred to in Spanish standard method UNE-153601-22008.
- Methacrylate disk 90 mm in diameter by 10 mm thick
- Weight of 10 kg (with stand for its use). Total pressure exerted on the paper of the filter is 2,2 psi.
- Three decimal analytical balance.
- Liquid for testing: Saline solution: 0,9 %. 9 g ($\pm$ 0,1 g) of sodium chloride (NaCl) are weighed and dissolved in 1 l of distilled water.

**Test specifications**

Volume applied in all types of absorbent articles: apply 2 doses of 150 ml each

Application speed: 16 ml/s

Preparation and set-up of the sample

The absorbent article to be tested is kept for 2 hours at 23 ° C $\pm$ 2 and a relative humidity of 45-65 % before the test is begun.
The absorbent article to be tested is extended with the topsheet surface upward and all elements that might interfere with the test are put out of action. The absorbent article is kept completely extended (barriers, elastic between the legs, etc.). The absorbent article is extended and stretched out over a flat platform that makes it possible to move the sample without touching or handling the absorbent article. The entry point of the liquid is marked, and for that purpose the centre of the total length of the absorbent article is measured plus 2 cm toward the front part of the absorbent article and the centre of the width in the crotch of the absorbent centre (this point should not coincide with the transversal fold of the absorbent article, moving if necessary the entry point of the liquid).

Preparation of the equipment

1. The pear-shaped separatory funnel is fixed to the laboratory stand at a height sufficient for setting the methacrylate column below it (minimum 650 mm).
2. The separator tube and the weights are introduced into the methacrylate column so that all of it is supported by the platform below (see figure 20a and 20b).

3. The platform with the absorbent article to be tested is positioned extended under the pear-shaped separatory funnel with the absorbent part upward, leaving the crotch zone of the absorbent centre free.

4. The methacrylate column with the weights is positioned on top of the absorbent centre, so that the point marked coincide with the centre of the column, and the end of the funnel is introduced into the tube of the column. The absorbent article is gently stretched to eliminate possible wrinkles that could remain under the column.

5. 150 ml of saline solution is introduced into the pear-shaped separatory funnel funnel with the escape valve closed.

Performing the test

1. Once the test has been prepared in the way described, the unloading valve of the funnel is opened and at the same time chronometer 1 is put into operation and immediately afterwards chronometer 2.

2. Time passed (seconds) is measured in chronometer 1 from the time the valve is opened until the saline solution has totally penetrated the absorbent article. This value is saved (T1).

3. While the 15 minutes of the second chronometer pass, the valve of the pear-shaped separatory funnel funnel is closed again and another 150 ml of saline solution are introduced into the funnel.

4. After the 15 minutes have gone by, stop the second chronometer, open the discharge valve and at the same time start up chronometer 1 and immediately afterwards chronometer 2.

5. The time that has gone by (seconds) is measured from the time the valve is opened until the saline solution has totally penetrated the absorbent article. This value is saved (T2).

6. While the 15 minutes of the second chronometer go by, 40 disks of filter paper are prepared to measure dryness, they are weighed, and this value is saved (Ps). (See note 2.)

7. After the 15 minutes have gone by, the column is removed. Immediately afterward the platform or stand on which the absorbent article is set is taken and placed on the stand of the handling of the weight of 10 Kg. The absorbent article should not be handled.

8. The disks of filter paper are placed on the point marked and on top of the methacrylate disk of 90 mm in diameter, along with the 10 kg weight. The weight is maintained for 30 seconds (a mechanical device or similar apparatus should be used to raise or lower the weight).

9. When this time has passed (30 s) the weight and the disk are removed, the filter paper disks are collected and the value is saved (Pm).

**Calculation and expression of the results:**

The results of the Rewet test are expressed as:

Moisture return, unit grams:

$$R = P_m - P_s$$

In which

R = Value of moisture return in grams.
$P_s$ = Paper weight of dry filter.
$P_m$ = Paper weight of wet filter.

The rate of absorption under pressure can be calculated according to:

1. Rate of absorption 1; unit ml/s:

$$V_1 = A / T_1;$$

In which

$V_1$ = Rate of absorption / absorption in 1 dose.
A = Amount of dosified liquid (150 ml).
$T_1$ = Time passed in seconds.

2. Rate of absorption 2; unit ml/s:

$$V_2 = A / T_2 ;$$

In which

$V_2$= Rate of absorption / absorption in 1 dose.
A = Amount of dosified liquid (150 ml).
$T_2$ = Time passed in seconds.

The "ISO absorption capacity" of the absorbent article is determined by the maximum amount of liquid that can be absorbed by the article and is measured in grams [g] as described in accordance with ISO11948-1 (1996).

The "MD/CD tensile ratio" is the ratio of material's tensile strength at peak in the MD and CD direction. Both were measured according to the EDANA standard method WSP 110.4-R0 (15), where sample width is 50 mm, jaw distance is 100 mm, speed 100 mm/min and preload 0,1N. MD/CD ratio [-] = tensile strength at peak in MD [N/5cm] / tensile strength at peak in CD [N/5cm].

The "Thickness" or "Caliper" of the nonwoven fabrics is measured under a pressure of 0,5 kPa if not specified otherwise. The circular pressure foot shall have an area of 2500 mm$^2$. If the surface of the test sample is too small for this, a correspondingly smaller pressure foot shall be used which is also applied with a pressure of 0,5 kPa if not specified otherwise.

The "recovery" of the bulkiness after pressure herein refers to the ratio of thickness of the fabric after it was freed from the load to the original thickness of the fabric.

The recovery measurement procedure consists of following steps:

1. Measure the thickness of the fabric under a pressure of 0,5 kPa (Ts)
2. Apply a pressure of 2,5 kPa to the fabric for 5 minutes
3. Release the weight and wait for 5 minutes
4. Measure the thickness the fabric under a preassure of 0,5 kPa (Tr)
5. Calculate the recovery according to the following equation:

$$Recovery = Tr/Ts \ (no \ unit)$$

Ts = thickness of fresh sample
Tr = thickness of recovered sample

The **"compressibility"** herein refers to the distance in mm by which the nonwoven is compressed by the pressure defined in "resilience" measurement. It can be also calculated as resilience (no unit) *thickness (mm). The **"resilience"** of a nonwoven is measured according to the European standard test EN ISO 964-1 with following modification:

1. The thickness of the fabric is measured under a pressure of 0,5 kPa (T0).
2. A pressure of 2,5 kPa is applied with loading speed of 5 mm/min
3. The distance TI of the clamp movement is measured in mm
4. Resilience is calculated according to the equation:

$$R \ (no \ unit) = TI(mm) / T0(mm)$$

Or

$$R \ (\%) = TI(mm) / T0(mm) * 100\%$$

TI = distance of the clamp movement under the pressure of 0,5 kPa [mm] = how much was the fabric compressed
T0 = thickness under 0, 5 kPa[mm]

The **"length of the filament to the length of the fabric ratio"** can be measured in three different ways:

a) The length of the filaments is measured by stretching them out so that they extend along a line without exhibiting crimps

b) In a fabric bonded to a given level, it is not possible to use method a) to measure the length of the filaments, so that the following estimation may be used:

    a. A picture of the assessed layer is provided in such a magnification that the fibers can be well seen

    b. One single fiber is chosen and its path through the picture or at least through part of the picture is marked out

    c. The length of fiber marked out on the picture is measured to estimate its real length

    d. The length of the fabric, where the fiber is marked out is measured

    e. The estimated length of the filament to the length of the fabric ratio (percentage) is calculated

c) In a fabric using the **"Method for determining geometric fiber statistics for a nonwoven material",** where:

    a. The geometric representation of the fabric for analysis measures 8 mm in MD and 8 mm in CD, maintaining the full thickness of the sample in z-direction.
    b. Only the fibers, that enter the cropped sample volume on one side and leave it at opposite side are relevant for the measurement
    c. At least 20 filaments have to be measured
    d. The length of the filament to the length of the fabric ratio (percentage) is calculated

**"The type of fiber cross-section"** is known from the process conditions, defined by the fiber forming die. In the event that the process conditions are unknown, the following estimation can be used:
A sample of the fabric is taken and pictures of the cross-sections of at least 20 fibers are made. The cross-section is made on the free part of the fiber, not in the bonding point or in a place of contact with another fiber, where deformation can be expected. For each cross section, the component surface is marked out on the image separately for each component. The centre of mass is determined for each component based on the centroid or geometric center determination of the planar object and its position is recorded using the Cartesian coordinate system with the centre [O; O] in the geometrical centre of the fiber cross-section. The deflection (D) of centre of mass for each component in each fiber cross-section is calculated according to the following equation:

$$D = \text{absolute value } (x * y),$$

where x and y are the coordinates of centre of mass.
When one of the x, y values is equal to 0 and not the other, the sample is discarded from evaluation) The average value and standard deviation is calculated for each component.
The fiber is considered noncrimpable when the ((average deflection) plus (standard deviation)) to total fiber cross-section surface ratio is less than 5%.
The fiber is expected noncrimpable when the ratio ((average deflection) minus (standard deviation)) to total fiber cross-section surface ratio is less than 5%.
**"Median fiber diameter"** in a layer is expressed in SI units - micrometers ($\mu$m) or nanometers (nm). To determine the median, it is necessary to take a sample of the nonwoven fabric from at least three locations, preferably (if the sample is big enough) at least 5 cm away from each other. In each sample, it is necessary to measure the diameter of at least 50 individual fibers for each observed layer. It is possible to use, for example, an optical or electronic microscope (depending on the diameter of the measured fibers). In the event that the diameter of fibers in one sample varies significantly from the other two, it is necessary to discard the entire sample and to prepare a new one.
In the case of round fibers, the diameter is measured as a diameter of the cross-section of the fibers. In the event of any other shape of the fiber (e.g. hollow fiber or trilobal fiber), the cross-section surface shall be determined for each measured fiber and recalculated for a circle with same surface area. The diameter of this theoretical circle is the diameter of the fiber.
The measured values for each layer composed of all three samples are consolidated into a single set of values from which the median is subsequently determined. It applies that at least 50% of the fibers have a diameter less than or equal to the value of the median and at least 50% of the fibers have a diameter greater than or equal to the median. To identify the median of the given sample set of values, it is sufficient to arrange the values according to size and to take the value found in the middle of the list. In the event that the sample set has an even number of items, usually the median is determined as the arithmetic mean of the values in locations N/2 and N/2+1.

The "compressed void volume ratio" herein refers to the total amount of void space in a material relative to the bulk volume occupied by the material at a given pressure. The "compressed absolute void volume" refers to the volume of the void space (in $m^3$) per given weight of the fabric (in kg). Both under a given pressure as defined herein to simulate in use conditions.

The void volume can be calculated using the equation:

1.

Compressed void volume ratio [%] = 1-(basis weight [$g/m^2$] / (caliper [mm]*1000))/mass density [$kg/m^3$]) * 100%

2.

Compressed absolute void volume [$m^3/kg$] = 1 / (basis weight [$g/m^2$] *caliper [mm]) - 1/mass density [$kg/m^3$]

Where the mass density (fiber density) can be calculated from a known composition or measurement according to the norm ISO 1183-3:1999.

For webs made with multiple fiber types, the total fiber density (mass density) is the weight average of each individual fiber density:

fiber density, total = Wt % fiber 1 * mass density fiber I + Wt % fiber 2 * mass density fiber 2 + .....

where: wt % = weight percent of the fiber type in the web

It is understood herein that for purposes of measuring the caliper (thickness) of the fabric, the fabric sample is held under pressure of 2,5 kPa as referred to in the test for measuring "compressibility".

**Calculating "void volume softness index VVSI "**

The void volume softness index VVSI can be calculated using the equation:

void volume softness index VVSI = Compressed absolute void volume [$m^3/kg$] * structural softness [$m^4mm^2g^{-2}$ ].

**"Method to determine geometric fiber statistics for a nonwoven"**

In the following, we describe a software-based method to analyze a sample of a nonwoven material in order to characterize its geometric properties. The method uses a machine learning approach to identify the individual fibers present in the sample followed by a geometric analysis of these fibers to obtain statistics suitable for characterizing the material. The results include the orientation and density distribution of the fibers. This analysis workflow was developed by Math2Market GmbH and is part of the GeoDict digital material laboratory.

Step 1: Obtain three-dimensional $\mu$CT image of sample

First, the nonwoven sample is digitized using a $\mu$CT scanner to obtain a 3D image. The 3D image consists of a uniform Cartesian grid where each grid cell (Volume Element, Voxel) stores the X-Ray attenuation of the sample at the corresponding location. The pore space typically shows the lowest attenuation (smallest gray-scale value) while the material phase exhibits larger values, depending on the material and the configuration of the $\mu$CT device.

Step 2: Segment $\mu$CT image to separate material from pore space

For further analysis, the gray-scale image is noise-filtered using the Non-Local Means approach [1]. It is then binarized using a global threshold derived using Otsu's algorithm [2]. Binarization classifies each image voxel as containing either pore space or fiber material.

Voxels with gray values below the threshold are classified as pore space. All other voxels are classified as fiber material. For both operations, noise filtering and thresholding, the ImportGeo module of the GeoDict software is used.

Step 3: Analyze material density distribution

Furthermore, the material density distribution in z-direction is computed. For each slice of the image (at a given depth Z), the material density is computed as the number of white material voxels divided by the number of total voxels in the slice. This analysis is performed using the MatDict module of GeoDict.

Step 4: Apply a neural network to identify fiber centerlines

The main challenge in identifying individual fibers in $\mu$CT images is that, after binarization, the fibers are not spatially separated at contact points. This can result in under segmentation, where multiple objects (fibers) are erroneously classified as a single fiber.

To separate the fibers, Math2Market GmbH has developed an approach to identify the centerline curves of the fibers. These centerlines are represented in a binary voxel image of the same size as the original image. In this image, voxels within about 1-2 voxels to a fiber's center are marked.

For this purpose, we have used a semantic segmentation approach using a neural network [3]. The image is analyzed by considering a 3D sliding input window which is moved over the image. For each input window, a smaller output window is defined which is centered on the input window. The neural network analyzes the binary voxel values in the input window and produces a prediction for each voxel of the output window. The predicted value determines if a voxel inside the output window is part of a centerline. By combining the results for all these output windows we obtain a binary image which classifies each material voxel in the original image. This image transformation is implemented by the FiberFind-AI module in GeoDict, utilizing Tensorflow [4].

Step 5: Create training data for the neural networks

For the purpose of training the neural network to implement the transformation described above, Math2Market GmbH has created several artificial 3D images of nonwoven materials using the stochastic FiberGeo structure generation module in GeoDict. This module generates an analytical geometric representation of fibers as a series of line segments. At the same time, it outputs a binary image of the fiber structure, comparable to the binarization result of Step 2.

By modifying the fiber diameter in the analytical representation to about 2-3 voxels, we can likewise obtain an image of the centerlines corresponding to the artificial fiber structure.

These pairs of images (fibers and centerlines) are then used to train the neural network to transform a fiber image to a centerline image. The network effectively learns to "shrink" the fibers down to their centerline curves.

Step 6: Trace fiber centerlines to obtain geometric representation of fibers

After reducing the fibers to their centerlines, we assume that the centerlines do not touch. We then separate the individual centerlines from each other by analyzing the connected components of the centerline image, under the assumption that each component corresponds to the centerline of a single fiber. A connected component is defined as a subset of material voxels that all have the same color and that cannot be enlarged by adding any touching voxels of the same color. For each centerline, we trace a curve through the set of voxels to obtain a geometric representation of the corresponding fiber in the form of a sequence of connected line segments (a polyline). This step is also part of FiberFind-AI in GeoDict.

Step 7: Compute orientation distribution histogram of fibers

To obtain the orientation distribution in any plane (say, the XV plane), we first project each fiber line segment into that plane and compute the angle within the plane. Then, the orientation histogram is computed over the angle of all segments. Finally, this orientation histogram is visualized using a polar plot where the radius at a given angle is proportional to the frequency of occurrence of the corresponding orientation. This analysis is repeated for the remaining two planes (XZ and YZ).

[1] Buades, Antoni, Bartomeu Coll, and J-M. More!. "A non-local algorithm for image denoising." Computer Vision and Pattern Recognition, 2005. CVPR 2005. IEEE Computer Society Conference on. Vol. 2. IEEE, 2005.

[2] Otsu, Nobuyuki. "A threshold selection method from gray-level histograms." IEEE transactions on systems, man, and cybernetics 9.1 (1979): 62-66.

[3] Noh, Hyeonwoo, Seunghoon Hong, and Bohyung Han. "Learning deconvolution network for semantic segmentation." Proceedings of the IEEE international conference on computer vision. 2015.

[4] Martin Abadi, Ashish Agarwal, Paul Barharn, Eugene Brevdo, Zhifeng Chen, Craig Citro, Greg S. Corrado, Andy Davis, Jeffrey Dean, Matthieu Devin, Sanjay Ghemawat, Ian Goodfellow, Andrew Harp, Geoffrey Irving, Michael Isard, Rafal Jozefowicz, Yangqing Jia, Lukasz Kaiser, Manjunath Kudlur, Josh Levenberg, Dan Mane, Mike Schuster, Rajat Monga, Sherry Moore, Derek Murray, Chris Olah, Jonathan Shlens, Benoit Steiner, Ilya Sutskever, Kunal Talwar, Paul Tucker, Vincent Vanhoucke, Vijay Vasudevan, Fernanda Viegas, Oriol Vinyals, Pete Warden, Martin Wattenberg, Martin Wicke, Yuan Yu, and Xiaoqiang Zheng. "TensorFlow: Large-scale machine learning on heterogeneous systems", 2015. Software available from tensorflow.org.

**Claims**

1. An absorbent article, comprising an absorbent storage core and an acquisition component, the acquisition component comprises a nonwoven acquisition fabric, wherein the nonwoven acquisition fabric is an air-through bonded nonwoven acquisition fabric, wherein the nonwoven acquisition fabric is a spunbond nonwoven, wherein the basis weight of the nonwoven acquisition fabric is 20-110 g/m$^2$, the nonwoven acquisition fabric comprises filaments, the filaments comprise a first polymeric material and a second polymeric material, the second polymeric material having its melting point lower than the first polymeric material, wherein the first polymeric material and/or the second polymeric material consists of or comprises as the majority component polymeric material selected from the group consisting of polyesters, polyolefins, polylactic acid, polyester copolymers, polylactide copolymers and blends thereof, and wherein the first polymeric material is different from the second polymeric material, wherein the second polymeric material extends in the longitudinal direction of the filaments and forms at least a part of the surface of the filaments, wherein all components of the filaments are arranged across the cross-section of the filaments in a non-crimpable configuration, that is the center of gravity of surfaces formed by a component across the fiber cross-section is located in substantially the same location as the center of gravity of surfaces of each of the other components, wherein the filaments have a median fiber diameter of 5-50 microns, and the nonwoven fabric comprises filament-to-filament bonds formed of the second polymeric material wherein the nonwoven acquisition fabric has a structural softness as defined herein of at least 80 m$^4$mm$^2$g$^{-2}$, wherein

$$Structural\ softness = \frac{thickness}{basis\ weight} \times recovery \times \frac{compressibility}{basis\ weight} \times 10^6$$

wherein thickness is the thickness of the nonwoven structure in mm, basis weight is the basis weight of the nonwoven structure in grams per square meter, recovery is the ratio (Tr)/(Ts), wherein (Ts) is the initial thickness of the nonwoven structure under a pressure of 0,5 kPa and (Tr) is the recovered thickness of the nonwoven structure measured after a 2,5 kPa load has been applied and afterwards released, compressibility is in mm the difference between the initial thickness of the nonwoven structure and the thickness of the nonwoven structure under a pressure of 2,5 kPa, wherein all characteristics to be measured according to the test methods are defined herein in the description, wherein the absorbent article comprises a length L1, the nonwoven acquisition fabric comprises a length L2, wherein L2<L1.

2. An absorbent article according to claim 1, wherein the nonwoven acquisition fabric has a structural softness as defined herein of at least 100 m$^4$mm$^2$g$^{-2}$, preferably at least 110 m$^4$mm$^2$g$^{-2}$, more preferably at least 120 m$^4$mm$^2$g$^{-2}$, more preferably at least 130 m$^4$mm$^2$g$^{-2}$, more preferably at least 140 m$^4$mm$^2$g$^{-2}$, most preferably at least 150 m$^4$mm$^2$g$^{-2}$.

3. An absorbent article according to any of the preceding claims, wherein the nonwoven acquisition fabric has a compressed absolute void volume as defined herein of at least 0,010 m$^3$/kg, preferably at least 0,013 m$^3$/kg, more preferably at least 0,016 m$^3$/kg, more preferably at least 0,019 m$^3$/kg, more preferably at least 0,022 m$^3$/kg, more preferably at least 0,025 m$^3$/kg, most preferably at least 0,028 m$^3$/kg.

4. An absorbent article according to any of the preceding claims, wherein the nonwoven acquisition fabric has a void volume softness index VVSI as defined herein of at least 0,50, preferably at least 1,00, more preferably at least 2,00, more preferably at least 3,00, more preferably at least 4,00, more preferably at least 5,00, most preferably at least 6,00.

5. The absorbent article according to any of the preceding claims, wherein 0,1 x L1 < L2 < 0,9 x L1.

6. The absorbent article according to any of the preceding claims, wherein the absorbent article comprises a topsheet and a backsheet, and the absorbent storage core is disposed between the nonwoven acquisition fabric and the backsheet.

7. The absorbent article according to claim 6, wherein the topsheet is integral with the nonwoven acquisition fabric.

8. The absorbent article according to any of the preceding claims, wherein the nonwoven acquisition fabric has an upper layer and a lower layer, wherein the upper layer is making up at least part of the body facing side of the absorbent article and the lower layer is facing the absorbent storage core.

9. The absorbent article according to any of the preceding claims, wherein the absorbent storage core comprises superabsorbent material and optionally fiber material, more preferably cellulose fiber material.

10. The absorbent article according to any of the preceding claims, wherein the MD/CD tensile ratio of the nonwoven acquisition fabric as defined herein is 0,50-4,00, preferably 0,75-3,00, more preferably 0,80-2,00, more preferably 0,85-1,50, more preferably 0,90-1,30, most preferably 1,00-1,20.

11. The absorbent article according to any of the preceding claims, wherein the basis weight of the nonwoven acquisition fabric is 25-100 $g/m^2$, more preferably 30-90 $g/m^2$, more preferably 40-80 $g/m^2$, most preferably 50-70 $g/m^2$.

12. The absorbent article according to any of the preceding claims, wherein the compressed void volume ratio of the nonwoven acquisition fabric as defined herein is 0,900-0,990, preferably 0,910-0,985, more preferably 0,920-0,980, more preferably 0,930-0,975, more preferably at least 0,940, most preferably at least 0,950.

13. The absorbent article according to any of the preceding claims wherein the filaments have a core/sheath structure, wherein the first polymeric material forms the core and the second polymeric material forms the sheath.

14. The absorbent article according to any of the preceding claims, wherein the absorbent article is an incontinence absorbent article, preferably an incontinence slip, an incontinence pant or an incontinence pad, more specifically preferably selected from the group consisting of a T-shape incontinence slip, an H-shape incontinence slip, an H-shape incontinence pant.

15. The absorbent article according to any of the preceding claims, wherein the absorbent article shows an ISO absorption capacity as defined herein of less than 300 g.

16. The absorbent article according to any of the preceding claims 1-14, wherein the absorbent article shows an ISO absorption capacity as defined herein of equal to or more than 300 g, more preferably more than 500 g, more preferably more than 700 g, more preferably more than 900 g, most preferably more than 1100 g.

17. The absorbent article according to any of the preceding claims, wherein the absorbent storage core comprises less than 90%, more preferably less than 80%, more preferably less than 70%, more preferably less than 60%, more preferably less than 50%, more preferably at least 10%, more preferably at least 20%, more preferably at least 30%, most preferably at least 40% by weight superabsorbent material (SAP).

18. The absorbent article according to any of the preceding claims, wherein the nonwoven acquisition fabric has a thickness measured at 0,5 kPa, said thickness is uniform over the entire extension of the nonwoven acquisition fabric.

19. The absorbent article according to any of the preceding claims, wherein the nonwoven acquisition fabric is a single, unitary piece.

20. The absorbent article according to any of the preceding claims, wherein at least a component of the absorbent storage core has a first area and a second area, the basis weight of the absorbent material of the first area is different from the basis weight of the absorbent material of the second area.

21. The absorbent article according to claim 20, wherein the second area is free of absorbent material.

22. The absorbent article according to any of the preceding claims 20-21, wherein the nonwoven acquisition fabric covers at least in part, preferably entirely the second area.

23. The absorbent article according to any of the preceding claims 20-22, wherein the second area comprises at least one channel, more preferably one single channel disposed at least in part, more preferably all the way along the longitudinal centerline of the absorbent article.

24. The absorbent article according to any of the preceding claims, wherein the nonwoven acquisition fabric contains at least 20% of fibres with a 'length of the filament to the length of the fabric' ratio higher than 120%, and at least 10% of fibres with a 'length of the filament to the length of the fabric' ratio higher than 150%, and at least 10% of fibres with a 'length of the filament to the length of the fabric' ratio lower than 250%.

25. The absorbent article according to any of the preceding claims, wherein the nonwoven acquisition fabric does include less than 20%, preferably less than 15%, more preferably less than 10%, more preferably less than 5%, more preferably less than 2% filaments having a crimpable cross-section, most preferably the nonwoven acquisition fabric does not include any filaments having a crimpable cross-section.

26. The absorbent article according to any of the preceding claims, wherein the combined rewet-basis weight index FRW calculated as FRW = basis weight of the nonwoven acquisition fabric [g/m$^2$] * rewet [g] of the absorbent article is less than 110, more preferably less than 90, more preferably less than 80, even more preferably less than 70.


**Patentansprüche**

1. Absorbierender Artikel, umfassend einen absorbierenden Speicherkern und eine Aufnahmekomponente, wobei die Aufnahmekomponente einen Vlies-Aufnahmestoff umfasst, wobei der Vlies-Aufnahmestoff ein luftdurchlässig gebundener Vlies-Aufnahmestoff ist, wobei der Vlies-Aufnahmestoff ein Spinnvlies ist, wobei das Flächengewicht des Vlies-Aufnahmestoffs 20 bis 110 g/m$^2$ beträgt, der Vlies-Aufnahmestoff Filamente umfasst, die Filamente ein erstes Polymermaterial und ein zweites Polymermaterial umfassen, das zweite Polymermaterial einen niedrigeren Schmelzpunkt als das erste Polymermaterial aufweist, wobei das erste Polymermaterial und/oder das zweite Polymermaterial aus einem Polymermaterial besteht oder als Hauptkomponente ein Polymermaterial umfasst, das ausgewählt ist aus der Gruppe bestehend aus Polyestern, Polyolefinen, Polymilchsäure, Polyestercopolymeren, Polylactidcopolymeren und Gemischen davon, und wobei das erste Polymermaterial vom zweiten Polymermaterial verschieden ist, wobei das zweite Polymermaterial in der Längsrichtung der Filamente verläuft und mindestens einen Teil der Oberfläche der Filamente bildet, wobei alle Komponenten der Filamente über den Querschnitt der Filamente in einer nicht kräuselbaren Konfiguration angeordnet sind, was bedeutet, dass der Schwerpunkt von Oberflächen, die durch eine Komponente über den Faserquerschnitt gebildet werden, im Wesentlichen an der gleichen Stelle wie der Schwerpunkt von Oberflächen jeder der anderen Komponenten angeordnet ist, wobei die Filamente einen Median-Faserdurchmesser von 5 bis 50 Mikrometer aufweisen und der Vliesstoff Filament-zu-Filament-Bindungen umfasst, die aus dem zweiten Polymermaterial gebildet sind, wobei der Vlies-Aufnahmestoff eine wie hierin definierte strukturelle Weichheit von mindestens 80 m$^4$mm$^2$g$^{-2}$ aufweist, wobei

$$Strukturelle\ Weichheit = \frac{Dicke}{Flächengewicht} \times Rückformung \times \frac{Kompressibilität}{Flächengewicht} \times 10^6$$

wobei Dicke die Dicke der Vliesstruktur in mm ist, Flächengewicht das Flächengewicht der Vliesstruktur in Gramm pro Quadratmeter ist, Rückformung das Verhältnis (Tr)/(Ts) ist, wobei (Ts) die Anfangsdicke der Vliesstruktur unter einem Druck von 0,5 kPa ist und (Tr) die rückgeformte Dicke der Vliesstruktur ist, die gemessen wird, nachdem eine Last von 2,5 kPa aufgebracht und anschließend entfernt worden ist, Kompressibilität in mm die Differenz zwischen der Anfangsdicke der Vliesstruktur und der Dicke der Vliesstruktur unter einem Druck von 2,5 kPa ist, wobei alle bei den Prüfverfahren zu messenden Eigenschaften hierin in der Beschreibung definiert sind, wobei der absorbierende Artikel eine Länge L1 umfasst, der Vlies-Aufnahmestoff eine Länge L2 umfasst, wobei L2<L1.

2. Absorbierender Artikel nach Anspruch 1, wobei der Vlies-Aufnahmestoff eine wie hierin definierte strukturelle Weichheit von mindestens 100 m$^4$mm$^2$g$^{-2}$, vorzugsweise mindestens 110 m$^4$mm$^2$g$^{-2}$, bevorzugter mindestens 120 m$^4$mm$^2$g$^{-2}$, bevorzugter mindestens 130 m$^4$mm$^2$g$^{-2}$, bevorzugter mindestens 140 m$^4$mm$^2$g$^{-2}$, am meisten bevorzugt mindestens 150 m$^4$mm$^2$g$^{-2}$, aufweist.

3. Absorbierender Artikel nach einem der vorstehenden Ansprüche, wobei der Vlies-Aufnahmestoff ein wie hierin definiertes komprimiertes absolutes Hohlraumvolumen von mindestens 0,010 m³/kg, vorzugsweise mindestens 0,013 m³/kg, bevorzugter mindestens 0,016 m³/kg, bevorzugter mindestens 0,019 m³/kg, bevorzugter mindestens 0,022 m³/kg, bevorzugter mindestens 0,025 m³/kg, am meisten bevorzugt mindestens 0,028 m³/kg, aufweist.

4. Absorbierender Artikel nach einem der vorstehenden Ansprüche, wobei der Vlies-Aufnahmestoff einen wie hierin definierten Hohlraumvolumen-Weichheitsindex VVSI von mindestens 0,50, vorzugsweise mindestens 1,00, bevorzugter mindestens 2,00, bevorzugter mindestens 3,00, bevorzugter mindestens 4,00, bevorzugter mindestens 5,00, am meisten bevorzugt mindestens 6,00, aufweist.

5. Absorbierender Artikel nach einem der vorstehenden Ansprüche, wobei 0,1 x L1 < L2 < 0,9 x L1.

6. Absorbierender Artikel nach einem der vorstehenden Ansprüche, wobei der absorbierende Artikel eine Deckschicht und eine Rückenschicht umfasst und der absorbierende Speicherkern zwischen dem Vlies-Aufnahmestoff und der Rückenschicht angeordnet ist.

7. Absorbierender Artikel nach Anspruch 6, wobei die Deckschicht integral mit dem Vlies-Aufnahmestoff ist.

8. Absorbierender Artikel nach einem der vorstehenden Ansprüche, wobei der Vlies-Aufnahmestoff eine obere Schicht und eine untere Schicht aufweist, wobei die obere Schicht mindestens einen Teil der körperzugewandten Seite des absorbierenden Artikels bildet und die untere Schicht dem absorbierenden Speicherkern zugewandt ist.

9. Absorbierender Artikel nach einem der vorstehenden Ansprüche, wobei der absorbierende Speicherkern superabsorbierendes Material und gegebenenfalls Fasermaterial, bevorzugter Cellulosefasermaterial, umfasst.

10. Absorbierender Artikel nach einem der vorstehenden Ansprüche, wobei das wie hierin definierte MD/CD-Zugverhältnis des Vlies-Aufnahmestoffs 0,50 bis 4,00, vorzugsweise 0,75 bis 3,00, bevorzugter 0,80 bis 2,00, bevorzugter 0,85 bis 1,50, bevorzugter 0,90 bis 1,30, am meisten bevorzugt 1,00 bis 1,20, beträgt.

11. Absorbierender Artikel nach einem der vorstehenden Ansprüche, wobei das Flächengewicht des Vlies-Aufnahmestoffs 25 bis 100 g/m², bevorzugter 30 bis 90 g/m², bevorzugter 40 bis 80 g/m², am meisten bevorzugt 50 bis 70 g/m², beträgt.

12. Absorbierender Artikel nach einem der vorstehenden Ansprüche, wobei das wie hierin definierte Verhältnis des komprimierten Hohlraumvolumens des Vlies-Aufnahmestoffs 0,900 bis 0,990, vorzugsweise 0,910 bis 0,985, bevorzugter 0,920 bis 0,980, bevorzugter 0,930 bis 0,975, bevorzugter mindestens 0,940, am meisten bevorzugt mindestens 0,950, beträgt.

13. Absorbierender Artikel nach einem der vorstehenden Ansprüche, wobei die Filamente eine Kern/Mantel-Struktur aufweisen, wobei das erste Polymermaterial den Kern bildet und das zweite Polymermaterial den Mantel bildet.

14. Absorbierender Artikel nach einem der vorstehenden Ansprüche, wobei der absorbierende Artikel ein inkontinenzabsorbierender Artikel, vorzugsweise ein Inkontinenz-Slip, ein Inkontinenzhöschen oder ein Inkontinenz-Pad, insbesondere vorzugsweise ausgewählt aus der Gruppe bestehend aus einem T-förmigen Inkontinenz-Slip, einem H-förmigen Inkontinenz-Slip, einem H-förmigen Inkontinenzhöschen, ist.

15. Absorbierender Artikel nach einem der vorstehenden Ansprüche, wobei der absorbierende Artikel eine wie hierin definierte ISO-Absorptionskapazität von weniger als 300 g aufweist.

16. Absorbierender Artikel nach einem der vorstehenden Ansprüche 1 bis 14, wobei der absorbierende Artikel eine wie hierin definierte ISO-Absorptionskapazität von gleich oder mehr als 300 g, bevorzugter mehr als 500 g, bevorzugter mehr als 700 g, bevorzugter mehr als 900 g, am meisten bevorzugt mehr als 1100 g, aufweist.

17. Absorbierender Artikel nach einem der vorstehenden Ansprüche, wobei der absorbierende Speicherkern weniger als 90 Gew.-%, bevorzugter weniger als 80 Gew.-%, bevorzugter weniger als 70 Gew.-%, bevorzugter weniger als 60 Gew.-%, bevorzugter weniger als 50 Gew.-%, bevorzugter mindestens 10 Gew.-%, bevorzugter mindestens 20 Gew.-%, bevorzugter mindestens 30 Gew.-%, am meisten bevorzugt mindestens 40 Gew.-%, an superabsorbierendem Material (SAP) umfasst.

**18.** Absorbierender Artikel nach einem der vorstehenden Ansprüche, wobei der Vlies-Aufnahmestoff eine bei 0,5 kPa gemessene Dicke aufweist, wobei die Dicke über die gesamte Ausdehnung des Vlies-Aufnahmestoffs gleichmäßig ist.

**19.** Absorbierender Artikel nach einem der vorstehenden Ansprüche, wobei der Vlies-Aufnahmestoff ein einziges unitäres Stück ist.

**20.** Absorbierender Artikel nach einem der vorstehenden Ansprüche, wobei mindestens eine Komponente des absorbierenden Speicherkerns einen ersten Bereich und einen zweiten Bereich aufweist, wobei das Flächengewicht des absorbierenden Materials des ersten Bereichs vom Flächengewicht des absorbierenden Materials des zweiten Bereichs verschieden ist.

**21.** Absorbierender Artikel nach Anspruch 20, wobei der zweite Bereich frei von absorbierendem Material ist.

**22.** Absorbierender Artikel nach einem der vorstehenden Ansprüche 20 bis 21, wobei der Vlies-Aufnahmestoff den zweiten Bereich mindestens teilweise, vorzugsweise vollständig, bedeckt.

**23.** Absorbierender Artikel nach einem der vorstehenden Ansprüche 20 bis 22, wobei der zweite Bereich mindestens einen Kanal, bevorzugter einen einzigen Kanal, umfasst, der mindestens teilweise, bevorzugter vollständig, entlang der Längsmittellinie des absorbierenden Artikels angeordnet ist.

**24.** Absorbierender Artikel nach einem der vorstehenden Ansprüche, wobei der Vlies-Aufnahmestoff mindestens 20 % Fasern mit einem Verhältnis "Länge des Filaments zur Länge des Stoffs" von mehr als 120 %, und mindestens 10 % Fasern mit einem Verhältnis "Länge des Filaments zur Länge des Stoffs" von mehr als 150 %, und mindestens 10 % Fasern mit einem Verhältnis "Länge des Filaments zur Länge des Stoffs" von weniger als 250 % enthält.

**25.** Absorbierender Artikel nach einem der vorstehenden Ansprüche, wobei der Vlies-Aufnahmestoff weniger als 20 %, vorzugsweise weniger als 15 %, bevorzugter weniger als 10 %, bevorzugter weniger als 5 %, bevorzugter weniger als 2 %, Filamente mit einem kräuselbaren Querschnitt einschließt, wobei der Vlies-Aufnahmestoff am meisten bevorzugt keine Filamente mit einem kräuselbaren Querschnitt einschließt.

**26.** Absorbierender Artikel nach einem der vorstehenden Ansprüche, wobei der kombinierte Rückbenetzungs-Flächengewicht-Index FRW, berechnet als FRW = Flächengewicht des Vlies-Aufnahmestoffs [g/m$^2$] * Rückbenetzung [g] des absorbierenden Artikels, weniger als 110, bevorzugter weniger als 90, bevorzugter weniger als 80, noch bevorzugter weniger als 70, beträgt.

**Revendications**

**1.** Article absorbant, comprenant une âme de stockage absorbante et un composant d'acquisition, le composant d'acquisition comprenant un non-tissé d'acquisition, le non-tissé d'acquisition étant un non-tissé d'acquisition lié par air traversant, le non-tissé d'acquisition étant un non tissé filé-lié, la masse surfacique du non-tissé d'acquisition étant de 20-110 g/m$^2$, le non-tissé d'acquisition comprenant des filaments, les filaments comprenant un premier matériau polymère et un deuxième matériau polymère, le point de fusion du deuxième matériau polymère étant inférieur à celui du premier matériau polymère, le premier matériau polymère et/ou le deuxième matériau polymère consistant en ou comprenant comme constituant majoritaire un matériau polymère choisi dans le groupe constitué par les polyesters, les polyoléfines, l'acide polylactique, les copolymères de polyester, les copolymères de polylactide et les mélanges de ceux-ci, et le premier matériau polymère étant différent du deuxième matériau polymère, le deuxième matériau polymère s'étendant dans la direction longitudinale des filaments et formant au moins une partie de la surface des filaments, tous les composants des filaments étant disposés sur la section transversale des filaments dans une configuration non crêpable, autrement dit le centre de gravité de surfaces formées par un composant sur la section transversale de la fibre étant situé sensiblement au même emplacement que le centre de gravité de surfaces de chacun des autres composants, les filaments présentant un diamètre médian de fibre de 5-50 micromètres, et le non-tissé comprenant des liaisons entre filaments constituées du deuxième matériau polymère, le non-tissé d'acquisition présentant une souplesse structurale telle que définie ici d'au moins 80 m$^4$mm$^2$g$^{-2}$, dans lequel

$$souplesse\ structurale = \frac{épaisseur}{masse\ surfacique} \times récupération \times \frac{compressibilité}{masse\ surfacique} \times 10^6$$

dans lequel l'épaisseur est l'épaisseur de la structure non tissée en mm, la masse surfacique est la masse surfacique de la structure non tissée en grammes par mètre carré, la récupération est le rapport (Tr)/(Ts), (Ts) étant l'épaisseur initiale de la structure non tissée sous une pression de 0,5 kPa et (Tr) étant l'épaisseur récupérée de la structure non tissée mesurée après qu'une charge de 2,5 kPa a été appliquée et ensuite relâchée, la compressibilité est, en mm, la différence entre l'épaisseur initiale de la structure non tissée et l'épaisseur de la structure non tissée sous une pression de 2,5 kPa, toutes les caractéristiques à mesurer selon les méthodes d'essai étant définies dans la description, l'article absorbant présentant une longueur L1, le non-tissé d'acquisition présentant une longueur L2, avec L2 < L1.

2.  Article absorbant selon la revendication 1, dans lequel le non-tissé d'acquisition présente une souplesse structurale telle que définie ici d'au moins 100 $m^4mm^2g^{-2}$, de préférence au moins 110 $m^4mm^2g^{-2}$, mieux au moins 120 $m^4mm^2g^{-2}$, mieux encore au moins 130 $m^4mm^2g^{-2}$, encore mieux au moins 140 $m^4mm^2g^{-2}$, idéalement au moins 150 $m^4mm^2g^{-2}$.

3.  Article absorbant selon l'une quelconque des revendications précédentes, dans lequel le non-tissé d'acquisition présente un volume de vide absolu sous compression tel que défini ici d'au moins 0,010 $m^3$/kg, de préférence au moins 0,013 $m^3$/kg, mieux au moins 0,016 $m^3$/kg, mieux encore d'au moins 0,019 $m^3$/kg, encore mieux au moins 0,022 $m^3$/kg, mieux encore au moins 0,025 $m^3$/kg, idéalement au moins 0,028 $m^3$/kg.

4.  Article absorbant selon l'une quelconque des revendications précédentes, dans lequel le non-tissé d'acquisition présente un indice de souplesse de volume de vide VVSI tel que défini ici d'au moins 0,50, de préférence au moins 1,00, mieux au moins 2,00, mieux encore au moins 3,00, encore mieux au moins 4,00, mieux encore au moins 5,00, idéalement au moins 6,00.

5.  Article absorbant selon l'une quelconque des revendications précédentes, dans lequel 0,1 x L1 < L2 < 0,9 x L1.

6.  Article absorbant selon l'une quelconque des revendications précédentes, l'article absorbant comprenant une feuille de dessus et une feuille de fond, et l'âme de stockage absorbante étant disposée entre le non-tissé d'acquisition et la feuille de fond.

7.  Article absorbant selon la revendication 6, dans lequel la feuille de dessus est solidaire du non-tissé d'acquisition.

8.  Article absorbant selon l'une quelconque des revendications précédentes, dans lequel le non-tissé d'acquisition présente une couche supérieure et une couche inférieure, la couche supérieure constituant au moins une partie du côté de l'article absorbant faisant face au corps et la couche inférieure faisant face à l'âme de stockage absorbante.

9.  Article absorbant selon l'une quelconque des revendications précédentes, dans lequel l'âme de stockage absorbante comprend un matériau superabsorbant et éventuellement un matériau fibreux, mieux encore un matériau fibreux cellulosique.

10. Article absorbant selon l'une quelconque des revendications précédentes, dans lequel le rapport de traction MD/CD du non-tissé d'acquisition tel que défini ici est de 0,50-4,00, de préférence 0,75-3,00, mieux 0,80-2,00, mieux encore 0,85-1,50, encore mieux 0,90-1,30, idéalement 1,00-1,20.

11. Article absorbant selon l'une quelconque des revendications précédentes, dans lequel la masse surfacique du non-tissé d'acquisition est de 25-100 $g/m^2$, mieux 30-90 $g/m^2$, mieux encore 40-80 $g/m^2$, idéalement 50-70 $g/m^2$.

12. Article absorbant selon l'une quelconque des revendications précédentes, dans lequel le taux de volume de vide sous compression du non-tissé d'acquisition tel que défini ici est de 0,900-0,990, de préférence 0,910-0,985, mieux 0,920-0,980, mieux encore 0,930-0,975, encore mieux au moins 0,940, idéalement au moins 0,950.

13. Article absorbant selon l'une quelconque des revendications précédentes dans lequel les filaments présentent une structure âme/gaine, le premier matériau polymère formant l'âme et le deuxième matériau polymère formant la gaine.

14. Article absorbant selon l'une quelconque des revendications précédentes, l'article absorbant étant un article absorbant pour l'incontinence, de préférence un slip d'incontinence, une culotte d'incontinence ou un tampon d'incontinence, plus spécifiquement choisi de préférence dans le groupe constitué par un slip d'incontinence en forme de T, un slip d'incontinence en forme de H, une culotte d'incontinence en forme de H.

15. Article absorbant selon l'une quelconque des revendications précédentes, l'article absorbant présentant une capacité d'absorption ISO telle que définie ici de moins de 300 g.

16. Article absorbant selon l'une quelconque des revendications 1 à 14 précédentes, l'article absorbant présentant une capacité d'absorption ISO telle que définie ici égale ou supérieure à 300 g, mieux supérieure à 500 g, mieux encore supérieure à 700 g, encore mieux supérieure à 900 g, idéalement supérieure à 1100 g.

17. Article absorbant selon l'une quelconque des revendications précédentes, dans lequel l'âme de stockage absorbante comprend moins de 90 %, de préférence moins de 80 %, mieux moins de 70 %, mieux encore moins de 60 %, encore mieux moins de 50 %, mieux au moins 10 %, mieux encore au moins 20 %, encore mieux au moins 30 %, idéalement au moins 40 % en poids de matériau superabsorbant (SAP).

18. Article absorbant selon l'une quelconque des revendications précédentes, dans lequel le non-tissé d'acquisition présente une épaisseur mesurée à 0,5 kPa, ladite épaisseur étant uniforme sur l'étendue entière du non-tissé d'acquisition.

19. Article absorbant selon l'une quelconque des revendications précédentes, dans lequel le non-tissé d'acquisition est une pièce unique, unitaire.

20. Article absorbant selon l'une quelconque des revendications précédentes, dans lequel au moins un composant de l'âme de stockage absorbante présente une première zone et une deuxième zone, la masse surfacique du matériau absorbant de la première zone étant différente de la masse surfacique du matériau absorbant de la deuxième zone.

21. Article absorbant selon la revendication 20, dans lequel la deuxième zone est dépourvue de matériau absorbant.

22. Article absorbant selon l'une quelconque des revendications 20 à 21 précédentes, dans lequel le non-tissé d'acquisition recouvre au moins en partie, de préférence entièrement la deuxième zone.

23. Article absorbant selon l'une quelconque des revendications 20 à 22 précédentes, dans lequel la deuxième zone comprend au moins un canal, mieux encore un seul canal disposé le long d'au moins en partie, mieux encore de la totalité de l'axe longitudinal de l'article absorbant.

24. Article absorbant selon l'une quelconque des revendications précédentes, dans lequel le non-tissé d'acquisition contient au moins 20 % de fibres avec un rapport 'entre longueur du filament et longueur du tissu' supérieur à 120 %, et au moins 10 % de fibres avec un rapport 'entre longueur du filament et longueur du tissu' supérieur à 150 %, et au moins 10 % de fibres avec un rapport 'entre longueur du filament et longueur du tissu' inférieur à 250 %.

25. Article absorbant selon l'une quelconque des revendications précédentes, dans lequel le non-tissé d'acquisition comporte moins de 20 %, de préférence moins de 15 %, mieux moins de 10 %, mieux encore moins de 5 %, encore mieux moins de 2 % de filaments présentant une section transversale crêpable, idéalement le non-tissé d'acquisition ne comporte aucun filament présentant une section transversale crêpable.

26. Article absorbant selon l'une quelconque des revendications précédentes, dans lequel l'indice de masse surfacique après réhumidification combiné FRW calculé comme FRW = masse surfacique du non-tissé d'acquisition [g/m$^2$] * réhumidification [g] de l'article absorbant est inférieur à 110, mieux inférieur à 90, mieux encore inférieur à 80, encore mieux inférieur à 70.

Fig. 1

Fig. 2

Fig. 3

Fig. 4

Fig. 5

Fig. 6

Fig. 7

**Fig. 8**

**Fig. 9**

**Fig. 10**

Orientation in XY plane

105°  90°  75°
120°           60°
135°                45°
150°                   30°
165°                      15°
180°                        0°
195°                       345°
210°                    330°
225°                 315°
240°              300°
255°  270°  285°

**Fig. 11**

| after activation (oven, 120°C) | |
|---|---|
| *Example 2F: PET/PP* | *Example 4: PET/PET* |
| *MD change: -15%*<br>*CD change: - 15 %*<br>*Thickness change: + 103%*<br>*Volume change: + 47%*<br>*Soft* | *MD change: - 63%*<br>*CD change: - 63 %*<br>*Thickness change: + 222%*<br>*Volume change: - 56%*<br>*Hard* |
| | |

**Fig. 12**

| Before activation | After activation |
|---|---|
| | |

Fig. 13 a

CD  MD  Z

CD  MD

CD  Z

Z  MD

EP 3 811 917 B1

Fig. 13 b

Fig. 13 c

**Fig. 14**

Micrographs of rayon fibers (7.8X); A = no crimp, B = medium crimp (5.8 crimps/cm), C = high crimp (7.6 crimps cm)

Fig. 15

Fig. 16

Fig. 17

Fig. 18a

Fig. 18b

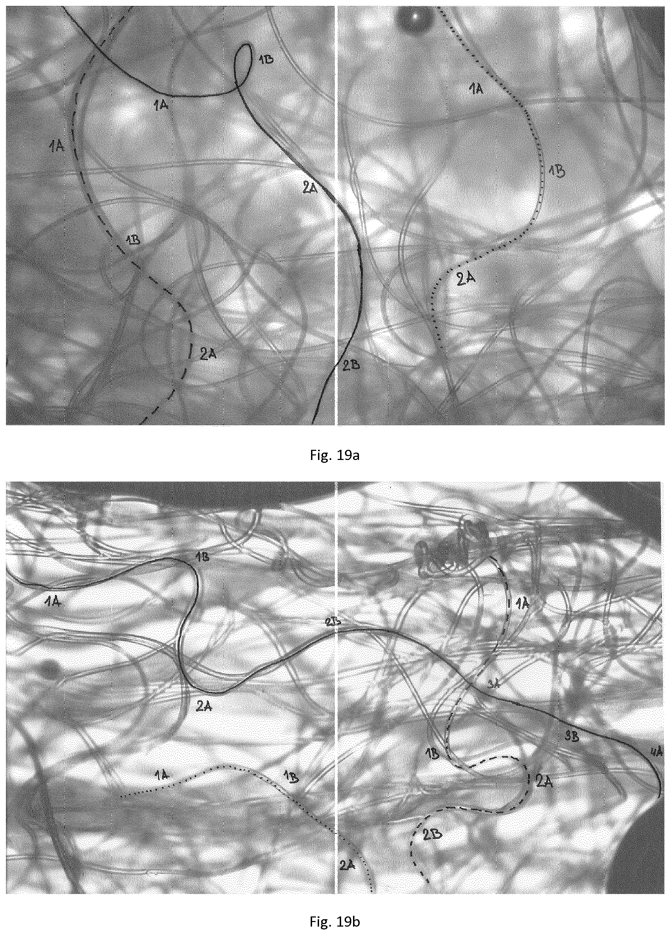

Fig. 19a

Fig. 19b

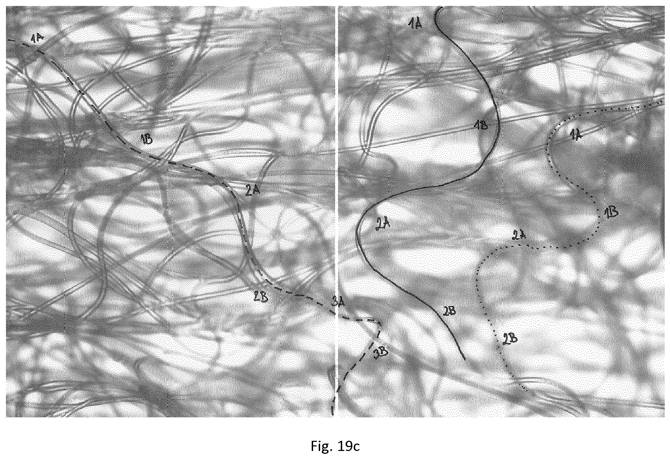

Fig. 19c

650

10
2

(a = 3,14

Ø107 mm    (a = 89,92

Figure 20a

Figure 20b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9111163 A1
- WO 2018059610 A1
- WO 2014022988 A
- WO 2015066299 A
- US 5753736 A
- US 20050159720 A1
- US 7112621 B1
- WO 02064877 A1
- US 6632385 B1
- US 6803103 B1

- US 0057921 A1
- US 2012088424 A
- WO 2009145105 A1
- WO 2017114695 A1
- EP 1763329 A1
- EP 2410965 A1
- EP 1906901 A1
- WO 2004016207 A2
- EP 1572057 A1
- EP 2849706 A1

### Non-patent literature cited in the description

- A non-local algorithm for image denoising. **BUADES** ; **ANTONI** ; **BARTOMEU COLL** ; **J-M. MORE**. Computer Vision and Pattern Recognition, 2005. CVPR 2005. IEEE Computer Society Conference on. IEEE, 2005, vol. 2
- **NOH** ; **HYEONWOO** ; **SEUNGHOON HONG** ; **BOHYUNG HAN**. Learning deconvolution network for semantic segmentation. *Proceedings of the IEEE international conference on computer vision*, 2015

- **MARTIN ABADI, ASHISH AGARWAL** ; **PAUL BARHARN, EUGENE BREVDO** ; **ZHIFENG CHEN, CRAIG CITRO** ; **GREG S. CORRADO, ANDY DAVIS** ; **JEFFREY DEAN, MATTHIEU DEVIN** ; **SANJAY GHEMAWAT, LAN GOODFELLOW** ; **ANDREW HARP, GEOFFREY IRVING** ; **MICHAEL ISARD, RAFAL JOZEFOWICZ** ; **YANGQING JIA, LUKASZ KAISER** ; **MANJUNATH KUDLUR, JOSH LEVENBERG**. *TensorFlow: Large-scale machine learning on heterogeneous systems*, 2015